(19) Europäisches Patentamt / European Patent Office / Office européen des brevets

(11) EP 2 655 310 B1

(12) # EUROPÄISCHE PATENTSCHRIFT

(45) Veröffentlichungstag und Bekanntmachung des Hinweises auf die Patenterteilung:
**05.11.2014 Patentblatt 2014/45**

(21) Anmeldenummer: **11801704.5**

(22) Anmeldetag: **15.12.2011**

(51) Int Cl.:
***C07C 51/15*** *(2006.01)* ***C07C 53/02*** *(2006.01)*

(86) Internationale Anmeldenummer:
**PCT/EP2011/072945**

(87) Internationale Veröffentlichungsnummer:
**WO 2012/084691 (28.06.2012 Gazette 2012/26)**

(54) **VERFAHREN ZUR HERSTELLUNG VON AMEISENSÄURE DURCH UMSETZUNG VON KOHLENDIOXID MIT WASSERSTOFF**

METHOD FOR PRODUCING FORMIC ACID BY REACTING CARBON DIOXIDE WITH HYDROGEN

PROCÉDÉ DE PRODUCTION D'ACIDE FORMIQUE PAR MISE EN RÉACTION DE DIOXYDE DE CARBONE AVEC DE L'HYDROGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2010 EP 10196351**

(43) Veröffentlichungstag der Anmeldung:
**30.10.2013 Patentblatt 2013/44**

(73) Patentinhaber: **BASF SE**
**67056 Ludwigshafen (DE)**

(72) Erfinder:
- **SCHAUB, Thomas**
  **67433 Neustadt (DE)**
- **FRIES, Donata Maria**
  **68199 Mannheim (DE)**
- **PACIELLO, Rocco**
  **67098 Bad Dürkheim (DE)**
- **MOHL, Klaus-Dieter**
  **68766 Hockenheim (DE)**
- **SCHÄFER, Martin**
  **67269 Grünstadt (DE)**
- **RITTINGER, Stefan**
  **68199 Mannheim (DE)**
- **DECKERT, Petra**
  **69245 Bammental (DE)**
- **BASSLER, Peter**
  **68519 Viernheim (DE)**

(56) Entgegenhaltungen:
**EP-A1- 0 181 078** **WO-A1-2008/116799**
**WO-A2-2010/149507**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 655 310 B1

Printed by Jouve, 75001 PARIS (FR)

## Beschreibung

Die Erfindung betrifft ein Verfahren zur Herstellung von Ameisensäure durch Umsetzung von Kohlendioxid mit Wasserstoff in einem Hydrierreaktor in Gegenwart eines Katalysators, enthaltend ein Element aus der 8, 9. oder 10. Gruppe des Periodensystems, eines tertiären Amins und eines polaren Lösungsmittels, unter Bildung von Ameisensäure/Amin-Addukten als Intermediaten, die anschließend thermisch gespalten werden.

Addukte aus Ameisensäure und tertiären Aminen können thermisch in freie Ameisensäure und tertiäres Amin gespalten werden und dienen daher als Intermediate in der Herstellung von Ameisensäure.

Ameisensäure ist ein wichtiges und vielseitig einsetzbares Produkt. Sie wird beispielsweise zum Ansäuern bei der Herstellung von Futtermitteln, als Konservierungsmittel, als Desinfektionsmittel, als Hilfsstoff in der Textil- und Lederindustrie, als Gemisch mit ihren Salzen zur Enteisung von Flugzeugen und Start- und Landebahnen sowie als Synthesebaustein in der chemischen Industrie verwendet.

Die genannten Addukte aus Ameisensäure und tertiären Aminen können auf verschiedene Art und Weise hergestellt werden, beispielsweise (i) durch direkte Umsetzung des tertiären Amins mit Ameisensäure, (ii) durch Hydrolyse von Methylformiat zu Ameisensäure in Gegenwart des tertiären Amins oder (iii) durch katalytische Hydratisierung von Kohlenmonoxid oder Hydrierung von Kohlendioxid zu Ameisensäure in Gegenwart des tertiären Amins. Das letztgenannte Verfahren der katalytischen Hydrierung von Kohlendioxid hat den besonderen Vorteil, dass Kohlendioxid in großen Mengen verfügbar und hinsichtlich seiner Quelle flexibel ist.

Großtechnisch aussichtsreich erscheint dabei vor allem die katalytische Hydrierung von Kohlendioxid in Gegenwart von Aminen (W. Leitner, Angewandte Chemie 1995, 107, Seiten 2391 bis 2405; P. G. Jessop, T. Ikariya, R. Noyori, Chemical Reviews 1995, 95, Seiten 259 bis 272). Die hierbei gebildeten Addukte aus Ameisensäure und Aminen lassen sich thermisch in Ameisensäure und das eingesetzte, in die Hydrierung rückführbare Amin, spalten.

Der für die Reaktion notwendige Katalysator enthält ein oder mehrere Elemente aus der 8., 9. oder 10. Gruppe des Periodensystems, also Fe, Co, Ni, Ru, Rh, Pd, Os, Ir und/oder Pt. Bevorzugt enthält der Katalysator Ru, Rh, Pd, Os, Ir und/oder Pt, besonders bevorzugt Ru, Rh und/oder Pd und ganz besonders bevorzugt Ru.

Um ein wirtschaftliches Verfahren zu ermöglichen, muss der verwendete Katalysator aus zwei Gründen möglichst vollständig aus dem Produktstrom abgetrennt und in die Reaktion zurückgeführt werden:

(1) große Verluste des teuren Katalysators würden erhebliche Zusatzkosten verursachen und wären für einen wirtschaftlichen Betrieb des Verfahrens prohibitiv.

(2) bei der thermischen Spaltung der Ameisensäure/Amin-Addukte sollte möglichst wenig Katalysator vorliegen, da dieser unter Abwesenheit eines $CO_2$- und/oder $H_2$-Druckes auch die Rückreaktion katalysiert und damit zu Verlusten an der gebildeten Ameisensäure führt.

$$CO_2 + H_2 + NR_3 \overset{Kat.}{\rightleftarrows} x\ HCOOH{*}NR_3 \xrightarrow{\Delta,\ Vakuum} HCOOH + NR_3$$

Rückführung

Bildung von Ameisensäure durch $CO_2$-Hydrierung (x = 0.4 - 3)

$$x\ HCOOH{*}NR_3 \xrightarrow{Kat.\ \Delta,\ Vakuum} CO_2 + H_2 + NR_3$$

Zersetzung von Ameisensäure/Amin-Addukten unter Anwesenheit eines Katalysators (x = 0.4 - 3)

Die Übergangsmetall-katalysierte Zersetzung von Ameisensäure wurde vor allem in letzter Zeit ausführlich beschrieben: C. Fellay, N. Yan, P.J. Dyson, G. Laurenczy Chem. Eur. J. 2009, 15, 3752-3760; C. Fellay, P.J. Dyson, G. Laurenczy Angew. Chem. 2008, 120, 4030-4032; B. Loges, A. Boddien, H. Junge, M. Beller Angew. Chem. 2008, 120, 4026-4029; F. Joó ChemSusChem 2008, 1, 805-808; S. Enthaler ChemSusChem 2008, 1, 801-804; S. Fukuzumi, T. Kobayashi, T. Suenobu ChemSusChem 2008, 1, 827-834;A. Boddien, B. Loges, H. Junge, M. Beller ChemSusChem 2008, 1, 751-758.

Die hierbei eingesetzten Katalysatoren eignen sich grundsätzlich auch zur Hydrierung von $CO_2$ zu Ameisensäure (P.G. Jessop, T. Ikariya, R. Noyori Chem. Rev. 1995, 95, 259-272; P.G. Jessop, F. Joó, C.C. Tai Coord. Chem. Rev. 2004, 248, 2425-2442; P.G. Jessop, Homogeneous Hydrogenation of Carbon Dioxide, in: The Handbook of Homogeneous Hydrogenation, Hrsg.: J.G. de Vries, C.J. Elsevier, Volume 1, 2007, Wiley-VCH, S. 489-511). Somit müssen

die Hydrierkatalysatoren vor der thermischen Spaltung abgetrennt werden, um die unerwünschte Ameisensäurezersetzung zu unterbinden.

WO 2008/116.799 offenbart ein Verfahren zur Hydrierung von Kohlendioxid in Gegenwart eines in Lösung suspendierten oder homogen gelösten Katalysators enthaltend ein Übergangsmetall der VIII. Nebengruppe (Gruppe 8, 9, 10), eines tertiären Amins mit mindestens einer Hydroxylgruppe und eines polaren Lösungsmittels zu einem Addukt aus Ameisensäure und dem tertiären Amin. Durch die Hydroxylgruppe(n) im tertiären Amin wird gegenüber dem sonst üblicherweise eingesetzten Triethylamin eine erhöhte Kohlendioxid-Löslichkeit erreicht. Als bevorzugte Homogenkatalysatoren sind $RuH_2L_4$ mit einzähnigen Phosphor-basierten Liganden L und $RuH_2(LL)_2$ mit zweizähnigen Phosphor-basierten Liganden LL und als besonders bevorzugt $RuH_2[P(C_6H_5)_3]_4$ genannt. Als polare Lösungsmittel sind Alkohole, Ether, Sulfolane, Dimethylsulfoxid und Amide, deren Siedepunkt bei Normaldruck mindestens 5°C oberhalb dem von Ameisensäure liegt, genannt. Auch die bevorzugt einzusetzenden tertiären Amine weisen einen Siedepunkt oberhalb dem der Ameisensäure auf. Da keine Phasentrennung stattfindet, erfolgt die Aufarbeitung des gesamten Reaktionsaustrags durch Destillation, gegebenenfalls nach vorheriger Abtrennung des Katalysators, wobei dabei das gebildete Addukt aus Ameisensäure und dem tertiären Amin thermisch gespalten und die freigesetzte Ameisensäure als Kopfprodukt gewonnen wird. Das Sumpfprodukt enthaltend tertiäres Amin, polares Lösungsmittel und gegebenenfalls Katalysator wird zur Hydrierstufe zurückgeführt.

Nachteilig an diesem Verfahren ist die Zuführung des gesamten flüssigen Reaktionsaustrags in die Vorrichtung zur thermischen Spaltung und Destillation, gegebenenfalls nach vorgeschalteter spezifischer Abtrennung des homogenen Katalysators durch einen separaten Verfahrensschritt wie beispielsweise einer Extraktions-, Adsorptions-oder Ultrafiltrationsstufe. Als Folge davon ist die Vorrichtung zur thermischen Spaltung und Destillation hinsichtlich der höheren Flüssigkeitsbelastung als auch hinsichtlich der spezifischeren Trenneigenschaften größer und komplexer auszulegen, was sich unter anderem auch in den Investitionskosten niederschlägt (zum Beispiel via Ingenieurleistung, Material, Flächenbedarf). Zudem bedingt die höhere Flüssigkeitsbelastung auch einen höheren Energiebedarf.

Die grundlegenden Arbeiten zur katalytischen Hydrierung von Kohlendioxid zu Ameisensäure wurden jedoch bereits in den 1970er und 1980er Jahren gemacht. Als Ausfluss davon dürften wohl auch die in EP 0 095 321 A, EP 0 151 510 A und EP 0 181 078 A zum Patent angemeldeten Verfahren der BP Chemicals Ltd zu verstehen sein. Alle drei Schriften beschreiben die Hydrierung von Kohlendioxid in Gegenwart eines homogenen Katalysators enthaltend ein Übergangsmetall der VIII. Nebengruppe (Gruppe 8, 9, 10), eines tertiären Amins und eines polaren Lösungsmittels zu einem Addukt aus Ameisensäure und dem tertiären Amin. Als bevorzugte Homogenkatalysatoren sind in EP 0 095 321 A und EP 0 181 078 A jeweils Ruthenium-basierte Carbonyl-, Halogenid- und/oder Triphenylphosphin-haltige Komplexkatalysatoren und in EP 0 151 510 A Rhodium/Phosphin-Komplexe genannt. Bevorzugte tertiäre Amine sind $C_1$-$C_{10}$-Trialkylamine, insbesondere die kurzkettigen $C_1$-$C_4$-Trialkylamine, sowie cyclische und/oder verbrückte Amine wie 1,8-Diazabicyclo[5.4.0]undec-7-en, 1,4-Diazabicyclo[2.2.2]octan, Pyridin oder Picoline. Die Hydrierung erfolgt bei einem Kohlendioxid-Partialdruck von bis zu 6 MPa (60 bar), einem Wasserstoff-Partialdruck von bis zu 25 MPa (250 bar) und einer Temperatur von etwa Raumtemperatur bis 200°C.

EP 0 095 321 A und EP 0 151 510 A lehren den Einsatz eines Alkohols als polares Lösungsmittel. Da jedoch primäre Alkohole zur Bildung von Ameisensäureestern (or-ganischen Formiaten) neigen, sind sekundäre Alkohole, insbesondere Isopropanol, bevorzugt. Zudem wird die Gegenwart von Wasser als vorteilhaft beschrieben. Gemäß den Beispielen aus EP 0 095 321 A erfolgt die Aufarbeitung des Reaktionsaustrags durch eine direkt nachfolgende zweistufige Destillation, bei der in der ersten Stufe die Leichtsieder Alkohol, Wasser, tertiäres Amin und in der zweiten Stufe unter Vakuumbedingungen das Addukt aus Ameisensäure und dem tertiären Amin über Kopf abgetrennt werden. EP 0 151 510 A lehrt ebenfalls eine destillative Aufarbeitung, jedoch unter Verweis auf EP 0 126 524 A mit nachfolgendem Austausch des tertiären Amins im destillativ abgetrennten Addukt vor dessen thermischer Spaltung durch eine schwächere, weniger flüchtige Stickstoffbase, um die nachfolgende thermische Spaltung zur Darstellung der freien Ameisensäure zu erleichtern, beziehungsweise erst möglich zu machen.

EP 0 181 078 A lehrt die gezielte Auswahl des polaren Lösungsmittels anhand von drei wesentlichen Kriterien, die gleichzeitig erfüllt sein müssen:

(i) der homogene Katalysator muss im polaren Lösungsmittel löslich sein;
(ii) das polare Lösungsmittel darf die Hydrierung nicht nachteilig beeinflussen; und
(iii) das gebildete Addukt aus Ameisensäure und dem tertiären Amin sollte leicht vom polaren Lösungsmittel abtrennbar sein.

Als besonders geeignete polare Lösungsmittel sind verschiedene Glykole und Phenylpropanole genannt.

Die Aufarbeitung des Reaktionsaustrags gemäß der Lehre von EP 0 181 078 A erfolgt derart, dass zunächst in einem Verdampfer die gasförmigen Komponenten (vor allem nicht umgesetzte Edukte Wasserstoff und Kohlendioxid) über Kopf und der homogene Katalysator gelöst im polaren Lösungsmittel über Sumpf abgetrennt und zur Hydrierstufe zurückgeführt werden. Aus der verbleibenden Flüssigphase enthaltend das Addukt aus Ameisensäure und dem tertiären

Amin, freies tertiäres Amin und gegebenenfalls Wasser wird dann anschließend das Addukt aus Ameisensäure und dem tertiären Amin abgetrennt und der verbleibende Teil der Flüssigphase enthaltend das freie tertiäre Amin und gegebenenfalls Wasser zur Hydrierstufe zurückgeführt. Die Abtrennung kann durch Destillation oder durch Phasentrennung des Zweiphasensystems (Dekantierung) erfolgen.

Eine weitere wesentliche Lehre aus EP 0 181 078 A ist der anschließende, zwingende Austausch des tertiären Amins im abgetrennten Addukt vor dessen thermischer Spaltung durch eine schwächere, weniger flüchtige Stickstoffbase, um die nachfolgende thermische Spaltung zur Darstellung der freien Ameisensäure zu erleichtern beziehungsweise erst möglich zu machen. Als besonders geeignete schwächere Stickstoffbasen sind Imidazolderivate wie beispielsweise 1-n-Butylimidazol genannt.

Nachteilig am Verfahren des EP 0 181 078 A ist die sehr aufwändige, vierstufige Aufarbeitung des Reaktionsaustrags durch

(i) Abtrennung der gasförmigen Komponenten sowie des homogenen Katalysators und des polaren Lösungsmittels in einem Verdampfer und Rückführung zur Hydrierstufe;
(ii) Abtrennung des Addukts aus Ameisensäure und dem tertiären Amin in einer Destillationskolonne oder einem Phasenscheider und Rückführung des verbleibenden Flüssigkeitsstroms zur Hydrierstufe;
(iii) Austausch des tertiären Amins im Addukt aus Ameisensäure und dem tertiären Amin durch eine schwächere, weniger flüchtige Stickstoffbase in einem Reaktionskessel mit aufgesetzter Destillationskolonne und Rückführung des freigesetzten tertiären Amins zur Hydrierstufe; und
(iv) thermische Spaltung des Addukts aus Ameisensäure und der schwächeren Stickstoffbase und Rückführung der freigesetzten schwächeren Stickstoffbase zur Basenaustauschstufe.

Ein weiterer, wesentlicher Nachteil am Verfahren des EP 0 181 078 A, sowie auch an den Verfahren der EP 0 095 321 A und EP 0 151 510 A ist die Tatsache, dass sich das Addukt aus Ameisensäure und dem tertiären Amin in Gegenwart des homogenen Katalysators bei der Aufarbeitung im Verdampfer zum Teil wieder in Kohlendioxid und Wasserstoff rückspaltet. In EP 0 329 337 A ist daher als Lösung des Problems die Zugabe eines Zersetzungsinhibitors vorgeschlagen, welcher den homogenen Katalysator reversibel inhibiert. Als bevorzugte Zersetzungsinhibitoren sind Kohlenmonoxid und Oxidationsmittel genannt. Nachteilig daran ist jedoch die Einführung weiterer Substanzen in das Gesamtverfahren und die Notwendigkeit, den inhibierten homogenen Katalysator vor dessen erneutem Einsatz wieder zu aktivieren.

Auch EP 0 357 243 A befasst sich mit dem Nachteil der teilweisen Rückspaltung des Addukts aus Ameisensäure und dem tertiären Amin im Verfahren des EP 0 181 078 A durch die gemeinsame Aufarbeitung des Reaktionsaustrags im Verdampfer. Das in EP 0 357 243 A vorgeschlagene Verfahren lehrt bei der katalytischen Hydrierung des Kohlendioxids zu einem Addukt aus Ameisensäure und tertiärem Amin den Einsatz eines homogenen Katalysators enthaltend ein Übergangsmetall der VIII. Nebengruppe (Gruppe 8, 9, 10), eines tertiären Amins und zweier unterschiedlicher Lösungsmittel, nämlich eines unpolaren und eines polaren, jeweils inerten Lösungsmittels, welche zwei nicht-mischbare flüssige Phasen bilden. Als unpolare Lösungsmittel sind aliphatische und aromatische Kohlenwasserstoffe, jedoch auch Phosphine mit aliphatischen und/oder aromatischen Kohlenwasserstoffresten genannt. Als polare Lösungsmittel sind Wasser, Glycerin, Alkohole, Polyole, Sulfolane oder deren Mischungen genannt, wobei Wasser bevorzugt ist. Im unpolaren Lösungsmittel löst sich der homogene Katalysator, im polaren Lösungsmittel das Addukt aus Ameisensäure und tertiärem Amin. Nach Beendigung der Reaktion werden beide Flüssigphasen separiert, beispielsweise durch Dekantierung, und die unpolare Phase enthaltend den homogenen Katalysator und das unpolare Lösungsmittel zur Hydrierstufe zurückgeführt. Die polare Phase enthaltend das Addukt aus Ameisensäure und tertiärem Amin und das polare Lösungsmittel wird dann einem zwingenden Austausch des tertiären Amins im Addukt vor dessen thermischer Spaltung durch eine schwächere, weniger flüchtige Stickstoffbase unter-worfen, um die nachfolgende thermische Spaltung zur Darstellung der freien Ameisensäure zu erleichtern beziehungsweise erst möglich zu machen. Analog EP 0 181 078 A sind auch hier Imidazolderivate wie beispielsweise 1-n-Butylimidazol als besonders geeignete schwächere Stickstoffbasen genannt.

Nachteilig am Verfahren des EP 0 357 243 A ist die sehr aufwändige, dreistufige Aufarbeitung des Reaktionsaustrags durch

(i) Trennung der beiden Flüssigphasen und Rückführung der den homogenen Katalysator und das unpolare Lösungsmittel enthaltenden Phase zur Hydrierstufe;
(ii) Austausch des tertiären Amins im Addukt aus Ameisensäure und dem tertiären Amin der anderen Phase durch eine schwächere, weniger flüchtige Stickstoffbase in einem Reaktionskessel mit aufgesetzter Destillationskolonne und Rückführung des freigesetzten tertiären Amins zur Hydrierstufe; und
(iii) thermische Spaltung des Addukts aus Ameisensäure und der schwächeren Stickstoffbase und Rückführung der freigesetzten schwächeren Stickstoffbase zur Basenaustauschstufe.

Ein weiterer Nachteil am Verfahren des EP 0 357 243 A ist der Einsatz zweier Lösungsmittel und somit die Einführung einer weiteren Substanz in das Gesamtverfahren.

Alternativ offenbart EP 0 357 243 A auch die Möglichkeit, nur ein Lösungsmittel einzusetzen. In diesem Fall entfällt die Zugabe des polaren Lösungsmittels, in dem sich ansonsten das Addukt aus Ameisensäure und dem tertiären Amin lösen würde. Das einzige hierbei eingesetzte Lösungsmittel ist das unpolare Lösungsmittel, welches den homogenen Katalysator löst. Auch bei dieser Alternative ist jedoch die sehr aufwändige, dreistufige Aufarbeitung wie oben beschrieben von Nachteil.

DE 44 31 233 A beschreibt ebenfalls die Hydrierung von Kohlendioxid in Gegenwart eines Katalysators enthaltend ein Übergangsmetall der VIII. Nebengruppe (Gruppe 8, 9, 10), eines tertiären Amins und eines polaren Lösungsmittels und Wasser zu einem Addukt aus Ameisensäure und dem tertiären Amin, bei dem jedoch der Katalysator heterogen vorliegt und die Aktivkomponente auf einem inerten Träger aufgebracht ist. Bevorzugte tertiäre Amine sind $C_1$-$C_8$-Trialkylamine, Polyamine mit 2 bis 5 Aminogruppen, aromatische Stickstoffheterocyclen wie Pyridin oder N-Methylimidazol, sowie cyclische und/oder verbrückte Amine wie N-Methylpiperidin, 1,8-Diazabicyclo[5.4.0]undec-7-en oder 1,4-Diazabicyclo[2.2.2]octan. Als geeignete polare Lösungsmittel sind die niedrig siedenden $C_1$-$C_4$-Monoalkohole genannt, wobei analog EP 0 095 321 A sekundäre Alkohole bevorzugt sind. Die Hydrierung erfolgt bei einem Gesamtdruck von 4 bis 20 MPa (40 bis 200 bar) und einer Temperatur von 50 bis 200°C. Zur Aufarbeitung des gebildeten Addukts aus Ameisensäure und tertiärem Amin lehrt DE 44 31 233 A den Einsatz bekannter Verfahren unter explizitem Verweis auf die in EP 0 357 243 A offenbarte Aufarbeitung unter Austausch des tertiären Amins im Addukt aus Ameisensäure und dem tertiären Amin durch eine schwächere, weniger flüchtige Stickstoffbase. Analog dem Verfahren nach EP 0 357 243 A ist auch beim Verfahren nach DE 44 31 233 A die sehr aufwändige, dreistufige Aufarbeitung des Reaktionsaustrags nachteilig.

Aufgabe der vorliegenden Erfindung war es, ein Verfahren zur Herstellung von Ameisensäure durch Hydrierung von Kohlendioxid zur Verfügung zu stellen, das die genannten Nachteile des Standes der Technik nicht oder nur in deutlich vermindertem Ausmaß aufweist und das zu konzentrierter Ameisensäure in hoher Ausbeute und hoher Reinheit führt. Ferner soll das Verfahren eine einfache oder zumindest einfachere Verfahrensführung erlauben, als im Stand der Technik beschrieben, insbesondere ein einfacheres Verfahrenskonzept zur Aufarbeitung des Austrags aus dem Hydrierreaktor, einfachere Verfahrensstufen, eine geringere Anzahl an Verfahrensstufen oder einfachere Apparate. Des Weiteren soll das Verfahren auch mit einem möglichst niedrigen Energiebedarf durchgeführt werden können. Insbesondere soll für das bislang nur unbefriedigend gelöste Problem der Rückführung des Katalysators eine effiziente Lösung angeboten werden unter gleichzeitiger Gewährleistung einer hohen Aktivität des Hydrierkatalysators.

Die Aufarbeitung des Austrags aus dem Hydrierreaktor soll insbesondere ausschließlich mit den im Verfahren bereits befindlichen Stoffen, ohne zusätzliche Hilfsstoffe, erfolgen, und diese sollen im Verfahren vollständig oder weitgehend vollständig recycliert werden.

Die Aufgabe wird gelöst durch ein Verfahren zur Herstellung von Ameisensäure durch Umsetzung von Kohlendioxid (1) mit Wasserstoff (2) in einem Hydrierreaktor (I) in Gegenwart

- eines Katalysators, enthaltend ein Element aus der 8., 9. oder 10. Gruppe des Periodensystems,
- eines tertiären Amins, enthaltend mindestens 12 Kohlenstoffatome pro Molekül, sowie
- eines polaren Lösungsmittels, enthaltend einen oder mehrere Monoalkohole, ausgewählt aus Methanol, Ethanol, Propanolen und Butanolen,

unter Bildung von Ameisensäure/Amin-Addukten als Intermediaten, die anschließend thermisch gespalten werden,
wobei ein tertiäres Amin eingesetzt wird, das einen um mindestens 5 °C höheren Siedepunkt als Ameisensäure aufweist, und wobei
sich bei der Umsetzung im Hydrierreaktor (I) ein Reaktionsgemisch enthaltend das polare Lösungsmittel, die Ameisensäure/Amin-Addukte, das tertiäre Amin und den Katalysator, bildet, das aus dem Reaktor als Austrag (3) ausgetragen wird,
dadurch gekennzeichnet, dass
der Austrag (3) aus dem Hydrierreaktor (I), gegebenenfalls nach Zugabe von Wasser, direkt einer Extraktionseinheit (II) zugeführt wird und die Aufarbeitung des Austrags (3) die folgenden Verfahrensschritte umfasst:

1) Extraktion des Katalysators aus dem Austrag (3) aus dem Hydrierreaktor (I) in der Extraktionseinheit (II), wobei als Extraktionsmittel das gleiche tertiäre Amin, das in der Hydrierung eingesetzt wurde, eingesetzt wird, unter Erhalt eines Extrakts (4), der den Hauptteil des tertiären Amins und des Katalysators enthält, und der in den Hydrierreaktor (I) recycliert wird sowie eines Raffinats (5), das den Hauptteil des polaren Lösungsmittels und der Ameisensäure/Amin-Addukte enthält, und das in eine Destillationseinheit (III) weitergeleitet wird, worin

2) eine Destillation durchgeführt wird unter Erhalt eines Kopfstroms (6), der überwiegend das polare Lösungsmittel

enthält, und der in den Hydrierreaktor (I) recycliert wird, sowie eines Sumpfstroms (7), der überwiegend die Ameisensäure/Amin-Addukte und das tertiäre Amin enthält, und welcher gemäß Verfahrensschritt

3) einem Phasentrenngefäß (IV) zugeführt wird, worin

a) eine Phasentrennung durchgeführt wird unter Erhalt einer Oberphase, enthaltend überwiegend das tertiäre Amin, die als Strom (11) in die Extraktionseinheit (II) als Extraktionsmittel für den Katalysator recycliert wird, und einer Unterphase, enthaltend überwiegend die Ameisensäure/Amin-Addukte, die als Strom (8) einer thermischen Spalteinheit (V) zugeführt wird, worin

b) eine thermische Spaltung durchgeführt wird unter Erhalt eines das tertiäre Amin enthaltenden Stroms (9), der in das Phasentrenngefäß (IV) zurückgeführt wird und eines die Ameisensäure enthaltenden Stroms (10), oder gemäß Verfahrensschritt

4) einer thermischen Spalteinheit (V) zugeführt wird, worin

a) eine thermische Spaltung durchgeführt wird unter Erhalt eines die Ameisensäure enthaltenden Stroms (10) und eines das tertiäre Amin und die Ameisensäure/Amin-Addukte enthaltenden Stroms (9), der einem Phasentrenngefäß (IV) zugeführt wird, worin

b) eine Phasentrennung durchgeführt wird unter Erhalt einer Oberphase, enthaltend überwiegend das tertiäre Amin, die als Strom (11) in die Extraktionseinheit (II) als Extraktionsmittel für den Katalysator recycliert wird, und einer Unterphase, enthaltend überwiegend die Ameisensäure/Amin-Addukte, die als Strom (8) einer thermischen Spalteinheit (V) zugeführt wird.

In einer Ausführungsform der vorliegenden Erfindung wird lediglich ein Teilstrom des das tertiäre Amin enthaltenden Stroms (11) aus dem Phasentrenngefäß (IV) in die Extraktionseinheit (II) als selektives Extraktionsmittel für den Katalysator geleitet und der verbleibende Teil des das tertiäre Amin enthaltenden Stroms direkt in den Hydrierreaktor (I) geleitet.

"Direkt einer Extraktionseinheit (II) zugeführt" im Rahmen der vorliegenden Erfindung bedeutet, dass der Austrag (3) aus dem Hydrierreaktor (I), gegebenenfalls nach Zugabe von Wasser, ohne weitere Aufarbeitungsschritte einer Extraktionseinheit (II) zugeführt wird.

Das erfindungsgemäße Verfahren umfasst in einer Ausführungsform daher die Verfahrensschritte 1), 2) und 3). In einer weiteren Ausführungsform umfasst das erfindungsgemäße Verfahren die Schritte 1), 2) und 4).

Der beim erfindungsgemäßen Verfahren bei der Hydrierung von Kohlendioxid einzusetzende Katalysator ist bevorzugt ein homogener Katalysator. Dieser enthält ein Element aus der 8., 9. oder 10. Gruppe des Periodensystems (nach IUPAC), also Fe, Co, Ni, Ru, Rh, Pd, Os, Ir und/oder Pt. Bevorzugt enthält der Katalysator Ru, Rh, Pd, Os, Ir und/oder Pt, besonders bevorzugt Ru, Rh und/oder Pd, und ganz besonders bevorzugt Ru.

Die genannten Elemente liegen in Form von komplexartigen Verbindungen im Reaktionsgemisch homogen gelöst vor. Der homogene Katalysator ist dabei so auszuwählen, dass dieser sich zusammen mit dem tertiären Amin in derselben Flüssigphase (B) anreichert. Unter "angereichert" ist dabei ein Verteilungskoeffizient des homogenen Katalysators

$$P = [\text{Konzentration homogener Katalysator in Flüssigphase (B)}] / [\text{Konzentration homogener Katalysator in Flüssigphase (A)}]$$

von > 1 zu verstehen. Die Auswahl des homogenen Katalysators erfolgt im Allgemeinen durch einen einfachen Versuch, indem der Verteilungskoeffizient des gewünschten homogenen Katalysators unter den geplanten Verfahrensbedingungen experimentell bestimmt wird.

Flüssigphase (A) ist hierbei das Raffinat in Verfahrensstufe 2).

Wegen ihrer guten Löslichkeit in tertiären Aminen verwendet man beim erfindungsgemäßen Verfahren als homogene Katalysatoren bevorzugt Komplexverbindungen, die ein Element aus der 8., 9. oder 10. Gruppe des Periodensystems und mindestens eine Phosphin-Gruppe mit mindestens einem unverzweigten oder verzweigten, acyclischen oder cyclischen, aliphatischen Rest mit 1 bis 12 Kohlenstoffatomen enthalten, wobei einzelne Kohlenstoffatome auch durch >P- substituiert sein können. Im Sinne von verzweigten cyclischen aliphatischen Resten sind damit eingeschlossen auch Reste wie beispielsweise $-CH_2-C_6H_{11}$. Als geeignete Reste sind beispielsweise genannt Methyl, Ethyl, 1-Propyl, 2-Propyl, 1-Butyl, 2-Butyl, 1-(2-Methyl)propyl, 2-(2-Methyl)propyl, 1-Pentyl, 1-Hexyl, 1-Heptyl, 1-Octyl, 1-Nonyl, 1-Decyl,

1-Undecyl, 1-Dodecyl, Cyclopentyl, Cyclohexyl, Cycloheptyl und Cyclooctyl, Methylcyclopentyl, Methylcyclohexyl, 1-(2-Methyl)-pentyl 1-(2-Ethyl)-hexyl, 1-(2-Propyl)heptyl und Norbonyl. Bevorzugt enthält der unverzweigte oder verzweigte, acyclische oder cyclische, aliphatische Rest mindestens 1 sowie bevorzugt maximal 10 Kohlenstoffatome. Im Falle eines ausschließlich cyclischen Restes im oben genannten Sinne beträgt die Zahl der Kohlenstoffatome 3 bis 12 und bevorzugt mindestens 4 sowie bevorzugt maximal 8 Kohlenstoffatome. Bevorzugte Reste sind Ethyl, 1-Butyl, sec-Butyl, 1-Octyl und Cyclohexyl.

Die Phosphin-Gruppe kann einen, zwei oder drei der oben genannten unverzweigten oder verzweigten, acyclischen oder cyclischen, aliphatischen Reste enthalten. Diese können gleich oder verschieden sein. Bevorzugt enthält die Phosphin-Gruppe drei der oben genannten unverzweigten oder verzweigten, acyclischen oder cyclischen, aliphatischen Reste, wobei besonders bevorzugt alle drei Reste gleich sind. Bevorzugte Phosphine sind $P(n\text{-}C_nH_{2n+1})_3$ mit n gleich 1 bis 10, besonders bevorzugt Tri-n-butylphosphin, Tri-n-octylphosphin und 1,2-Bis(dicyclohexylphosphino)ethan.

Wie bereits weiter oben erwähnt, können in den genannten unverzweigten oder verzweigten, acyclischen oder cyclischen, aliphatischen Resten einzelne Kohlenstoffatome auch durch >P- substituiert sein. Somit sind auch mehrzähnige, beispielsweise zwei- oder dreizähnige, Phosphin-Liganden mit umfasst. Diese enthalten bevorzugt die Gruppierung $>P\text{-}CH_2CH_2\text{-}P<$ beziehungsweise

$$>P\text{-}CH_2CH_2\text{-}\overset{|}{P}\text{-}CH_2CH_2\text{-}P<$$

Enthält die Phosphin-Gruppe noch andere Reste als die oben genannten unverzweigten oder verzweigten, acyclischen oder cyclischen, aliphatischen Reste, so entsprechen diese im Allgemeinen denen, die ansonsten üblicherweise bei Phosphin-Liganden für Komplexkatalysatoren eingesetzt werden. Als Beispiele seien genannt Phenyl, Tolyl und Xylyl.

Die Komplexverbindung kann eine oder mehrere, beispielsweise zwei, drei oder vier, der oben genannten Phosphin-Gruppen mit mindestens einem unverzweigten oder verzweigten, acyclischen oder cyclischen, aliphatischen Rest enthalten. Die restlichen Liganden des Komplexes können verschiedener Natur sein. Als exemplarische Beispiele seien genannt Hydrid, Fluorid, Chlorid, Bromid, Iodid, Formiat, Acetat, Propionat, Carboxylat, Acetylacetonat, Carbonyl, DMSO, Hydroxid, Trialkylamin, Alkoxid.

Die homogenen Katalysatoren können sowohl direkt in ihrer aktiven Form als auch ausgehend von üblichen Standardkomplexen wie beispielsweise $[M(p\text{-Cymene})Cl_2]_2$, $[M(\text{benzol})Cl_2]_n$, [M(COD)(allyl)], $[MCl_3xH_2O]$, $[M(\text{acetylacetonat})_3]$, $[M(DMSO)_4Cl_2]$ mit M gleich Element aus der 8., 9. oder 10. Gruppe des Periodensystems unter Zugabe des beziehungsweise der entsprechenden Phosphin-Liganden erst unter Reaktionsbedingungen erzeugt werden.

Beim erfindungsgemäßen Verfahren bevorzugte homogene Katalysatoren sind $[Ru(P^nBu_3)_4(H)_2]$, $(Ru(P^nOctyl_3)_4(H)_2]$, $[Ru(P^nBu_3)_2(1{,}2\text{-bis(dicyclohexylphosphino)-ethan})(H)_2]$, $[Ru(P^nOctyl_3)_2(1{,}2\text{-bis(dicyclohexylphosphino)ethan})(H)_2]$, $[Ru(PEt_3)_4(H)_2]$. Mit diesen können in der Hydrierung von Kohlendioxid TOF-Werte (turn-overfrequency) von größer 1000 $h^{-1}$ erzielt werden.

Beim Einsatz von homogenen Katalysatoren beträgt die eingesetzte Menge der genannten Metallkomponente im metallorganischen Komplex im Allgemeinen 0,1 bis 5000 Gew.-ppm, bevorzugt 1 bis 800 Gew.-ppm und besonders bevorzugt 5 bis 800 Gew.-ppm, jeweils bezogen auf das gesamte flüssige Reaktionsgemisch im Hydrierreaktor.

Der Verteilungskoeffizient des homogenen Katalysators bezogen auf die Menge an Ruthenium in der Aminphase und der das Ameisensäure/Amin-Addukt enthaltenden Produktphase ist nach der Hydrierung im Bereich von P größer 0,5, bevorzugtermaßen größer 1,0 und besonders bevorzugtermaßen größer 4.

Das beim erfindungsgemäßen Verfahren bei der Hydrierung von Kohlendioxid einzusetzende tertiäre Amin weist einen um mindestens 5°C höheren Siedepunkt als Ameisensäure auf. Dabei ist, wie üblich, bei der Angabe der relativen Lage von Siedepunkten von Verbindungen zueinander auf jeweils denselben Druck abzustellen. Das tertiäre Amin ist dabei so ausgewählt und mit dem polaren Lösungsmittel abgestimmt, dass das tertiäre Amin in der Oberphase im Hydrierreaktor angereichert vorliegt. Unter "angereichert" ist dabei ein Gewichtsanteil von > 50% des freien, das heißt nicht in Form des Ameisensäure/Amin-Addukts gebundenen, tertiären Amins in der Oberphase bezogen auf die Gesamtmenge an freiem, tertiärem Amins in beiden Flüssigphasen zu verstehen. Bevorzugt liegt der Gewichtsanteil bei > 90 %. Die Auswahl des tertiären Amins erfolgt im Allgemeinen durch einen einfachen Versuch, in dem die Löslichkeit des gewünschten tertiären Amins in beiden Flüssigphasen unter den geplanten Verfahrensbedingungen experimentell bestimmt wird. Die Oberphase kann zudem noch Teile des polaren Lösungsmittels sowie eines unpolaren inerten Lösungsmittel enthalten.

Bevorzugt weist das einzusetzende tertiäre Amin einen um mindestens 10°C, besonders bevorzugt einem um mindestens 50°C und ganz besonders bevorzugt einem um mindestens 100°C höheren Siedepunkt als Ameisensäure auf. Eine Einschränkung hinsichtlich eines oberen Grenzwertes für den Siedepunkt ist nicht erforderlich, da für das erfindungsgemäße Verfahren ein möglichst niedriger Dampfdruck des tertiären Amin grundsätzlich von Vorteil ist. Im

Allgemeinen liegt der Siedepunkt des tertiären Amins bei einem, gegebenenfalls nach bekannten Methoden vom Vakuum auf 1013 hPa abs hochgerechneten Druck unterhalb von 500°C.

Das beim erfindungsgemäßen Verfahren bevorzugt einzusetzende tertiäre Amin ist ein Amin, enthaltend mindestens 12 Kohlenstoffatome pro Molekül der allgemeinen Formel (Ia)

$$NR^1R^2R^3 \quad \text{(Ia),}$$

in der die Reste $R^1$ bis $R^3$ gleich oder verschieden sind und unabhängig voneinander einen unverzweigten oder verzweigten, acyclischen oder cyclischen, aliphatischen, araliphatischen oder aromatischen Rest mit jeweils 1 bis 16 Kohlenstoffatomen, bevorzugt 1 bis 12 Kohlenstoffatomen, darstellen, wobei einzelne Kohlenstoffatome unabhängig voneinander auch durch eine Heterogruppe ausgewählt aus der Gruppe -O-und >N- substituiert sein können sowie zwei oder alle drei Reste unter Bildung einer mindestens jeweils vier Atome umfassenden Kette auch miteinander verbunden sein können.

Als geeignete Amine seien beispielsweise genannt:

- Tri-n-butylamin, Tri-n-pentylamin, Tri-n-hexylamin, Tri-n-heptylamin, Tri-n-octylamin, Tri-n-nonylamin, Tri-n-decylamin, Tri-n-undecylamin, Tri-n-dodecylamin, Tri-n-tridecylamin, Tri-n-tetradecylamin, Tri-n-pentadecylamin, Tri-n-hexadecylamin, Tri-(2-ethylhexyl)amin.

- Dimethyldecylamin, Dimethyldodecylamin, Dimethyltetradecylamin, Ethyl-di-(2-propyl)amin ($Sdp_{1013\ hPa}$ = 127°C), Dioctyl-methylamin, Dihexylmethylamin.

- Tricyclopentylamin, Tricyclohexylamin, Tricycloheptylamin, Tricyclooctylamin und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.

- Dimethyl-cyclohexylamin, Methyl-dicyclohexylamin, Diethyl-cyclohexylamin, Ethyldicyclohexylamin, Dimethyl-cyclopentylamin, Methyl-dicyclopentylamin.

- Triphenylamin, Methyl-diphenylamin, Ethyl-diphenylamin, Propyl-diphenylamin, Butyl-diphenylamin, 2-Ethyl-hexyl-diphenylamin, Dimethyl-phenylamin, Diethylphenylamin, Dipropyl-phenylamin, Dibutyl-phenylamin, Bis(2-Ethyl-hexyl)-phenylamin, Tribenzylamin, Methyl-dibenzylamin, Ethyl-dibenzylamin und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.

- N-$C_1$-bis-$C_{12}$-Alkyl-piperidine, N,N-Di-$C_1$-bis-$C_{12}$-Alkyl-piperazine, N-$C_1$-bis-$C_{12}$-Alkyl-pyrrolidine, N-$C_1$-bis-$C_{12}$-Alkyl-imidazole und deren durch eine oder mehrere Methyl-, Ethyl-, 1-Propyl-, 2-Propyl-, 1-Butyl-, 2-Butyl- oder 2-Methyl-2-propyl-Gruppen substituierte Derivate.

- 1,8-Diazabicyclo[5.4.0]undec-7-en ("DBU"), 1,4-Diazabicyclo[2.2.2]octan ("DABCO"), N-Methyl-8-azabicyclo[3.2.1]octan ("Tropan"), N-Methyl-9-aza-bicyclo[3.3.1]nonan ("Granatan"), 1-Azabicyclo[2.2.2]octan ("Chinuclidin").

Beim erfindungsgemäßen Verfahren können natürlich auch Gemische einer beliebigen Anzahl verschiedener tertiärer Amine eingesetzt werden.

Besonders bevorzugt setzt man beim erfindungsgemäßen Verfahren als tertiäres Amin ein Amin der allgemeinen Formel (Ia), in der die Reste $R^1$ bis $R^3$ unabhängig voneinander ausgewählt sind aus der Gruppe $C_1$- bis $C_{12}$-Alkyl, $C_5$- bis $C_8$-Cycloalkyl, Benzyl und Phenyl, ein.

Besonders bevorzugt setzt man beim erfindungsgemäßen Verfahren als tertiäres Amin ein gesättigtes Amin, d.h. nur Einfachbindungen enthaltend, der allgemeinen Formel (Ia) ein.

Ganz besonders bevorzugt setzt man beim erfindungsgemäßen Verfahren als tertiäres Amin ein Amin der allgemeinen Formel (Ia) ein, in der die Reste $R^1$ bis $R^3$ unabhängig voneinander ausgewählt sind aus der Gruppe $C_5$- bis $C_8$-Alkyl, insbesondere Tri-n-pentylamin, Tri-n-hexylamin, Tri-n-heptylamin, Tri-n-octylamin, Dimethylcyclohexylamin, Methyldicyclohexylamin, Dioctyl-methylamin und Dimethyldecylamin.

Insbesondere setzt man als tertiäres Amin ein Amin der allgemeinen Formel (Ia) ein, in der die Reste $R^1$ bis $R^3$ unabhängig voneinander ausgewählt sind aus $C_5$- und $C_6$-Alkyl.

Bevorzugt liegt das tertiäre Amin beim erfindungsgemäßen Verfahren in allen Verfahrensstufen flüssig vor. Dies ist jedoch kein zwingendes Erfordernis. Es würde auch ausreichen, wenn das tertiäre Amin zumindest in geeigneten Lösungsmitteln gelöst wäre. Geeignete Lösungsmittel sind prinzipiell solche, welche hinsichtlich der Hydrierung von Kohlendioxid sowie der thermischen Spaltung des Addukts chemisch inert sind, in denen sich das tertiäre Amin und, im

Falle des Einsatzes eines homogenen Katalysators, auch diesen gut lösen, umgekehrt polares Lösungsmittel sowie das Ameisensäure/Amin-Addukte schlecht lösen. In Frage kommen daher prinzipiell chemisch inerte, unpolare Lösungsmittel wie etwa aliphatische, aromatische oder araliphatische Kohlenwasserstoffe, beispielsweise Octan und höhere Alkane, Toluol, Xylole.

Das beim erfindungsgemäßen Verfahren bei der Hydrierung von Kohlendioxid einzusetzende polare Lösungsmittel weist einen um mindestens 5°C niedrigeren Siedepunkt als die zur Spaltung der Ameisensäure/Amin-Addukte bei gleichem Druck benötigte Temperatur auf. Das polare Lösungsmittel ist dabei so auszuwählen beziehungsweise mit dem tertiären Amin abzustimmen, dass das polare Lösungsmittel in der Unterphase angereichert vorliegt. Unter "angereichert" ist dabei ein Gewichtsanteil von > 50% des polaren Lösungsmittels in der Unterphase bezogen auf die Gesamtmenge an polarem Lösungsmittel in beiden Flüssigphasen zu verstehen. Bevorzugt liegt der Gewichtsanteil bei > 70%. Die Auswahl des polaren Lösungsmittels erfolgt im Allgemeinen durch einen einfachen Versuch, in dem die Löslichkeit des gewünschten polaren Lösungsmittels in beiden Flüssigphasen unter den geplanten Verfahrensbedingungen experimentell bestimmt wird.

Bei dem polaren Lösungsmittel kann es sich um ein reines polares Lösungsmittel als auch um ein Gemisch verschiedener polarer Lösungsmittel handeln, solange die an das Lösungsmittel gestellten oben genannten Bedingungen bezüglich Siedepunkt und Phasenverhalten zutreffen.

Bevorzugt weist das einzusetzende polare Lösungsmittel einen um mindestens 10°C, besonders bevorzugt einen um mindestens 50°C niedrigeren Siedepunkt als die zur Spaltung der Ameisensäure/Amin-Addukte bei gleichem Druck benötigte Temperatur. Bei Lösungsmittelgemischen liegen die Siedpunkte des eingesetzten Lösungsmittelgemisches bzw. eines Azeotrops oder Heteroazeotrops um mindestens 10°C, besonders bevorzugt einem um mindestens 50°C niedriger als die zur Spaltung der Ameisensäure/Amin-Addukte bei gleichem Druck benötigte Temperatur.

Als Stoffklassen, die als polare Lösungsmittel geeignet sind, kommen bevorzugt Alkohole sowie deren Ameisensäureester und Wasser in Frage. Die Alkohole weisen einen um mindestens 10°C, besonders bevorzugt einem um mindestens 50°C niedrigeren Siedepunkt als die zur Spaltung der Ameisensäure/Amin-Addukte bei gleichem Druck benötigte Temperatur auf, um die Veresterung der Alkohole mit Ameisensäure möglichst gering zu halten.

Als geeignete Alkohole sind Alkohole genannt, in welchen sich im Gemisch mit Wasser die Trialkylammoniumformiate bevorzugt lösen und diese Produktphase eine Mischungslücke mit dem freien Trialkylamin aufweist. Als geeignete Alkohole seien beispielsweise Methanol, Ethanol, 2-Methoxyethanol, 1-Propanol, 2-Propanol, 1-Butanol, 2-Butano, 2-Methyl-1-propanol genannt. Als polares Lösungsmittel können auch Mischungen aus einem oder mehreren Alkoholen und Wasser eingesetzt werden. In einer Ausführungsform der Erfindung wird als polares Lösungsmittel ein Gemisch aus einem oder mehreren Monoalkoholen, ausgewählt aus Methanol, Ethanol, Propanolen und Butanolen mit Wasser eingesetzt. In einer weiteren Ausführungsform wird als polares Lösungsmittel ein Gemisch aus Methanol und/oder Ethanol mit Wasser eingesetzt. Das Verhältnis von Alkohol zu Wasser ist so zu wählen, das sich zusammen mit dem Trialkylammoniumformiat und dem Trialkylamin ein zweiphasiges Gemisch bildet, in welchem sich der Großteil des Trialkylammoniumformiates, das Wasser sowie das polare Lösungsmittel in der Unterphase befindet, was im Allgemeinen durch einen einfachen Versuch bestimmt wird, in dem die Löslichkeit des gewünschten polaren Lösungsmittelgemisches in beiden Flüssigphasen unter den geplanten Verfahrensbedingungen experimentell bestimmt wird.

Das Molverhältnis des beim erfindungsgemäßen Verfahren einzusetzenden polaren Lösungsmittels oder Lösungsmittelgemisches zum eingesetzten tertiären Amin beträgt im Allgemeinen 0,5 bis 30 und bevorzugt 1 bis 20.

In einer Ausführungsform der Erfindung wird dem Austrag (3) aus dem Hydrierreaktor (I) vor Zuführung zur Extraktionseinheit (II) Wasser zugegeben.

Gegenstand der Erfindung ist daher auch ein Verfahren zur Herstellung von Ameisensäure durch Umsetzung von Kohlendioxid (1) mit Wasserstoff (2) in einem Hydrierreaktor (I) in Gegenwart

- eines Katalysators, enthaltend ein Element aus der 8., 9. oder 10. Gruppe des Periodensystems,
- eines tertiären Amins, enthaltend mindestens 12 Kohlenstoffatome pro Molekül, sowie
- eines polaren Lösungsmittels, enthaltend einen oder mehrere Monoalkohole, ausgewählt aus Methanol, Ethanol, Propanolen und Butanolen,

unter Bildung von Ameisensäure/Amin-Addukten als Intermediaten, die anschließend thermisch gespalten werden, wobei ein tertiäres Amin eingesetzt wird, das einen um mindestens 5 °C höheren Siedepunkt als Ameisensäure aufweist, und wobei

sich bei der Umsetzung im Hydrierreaktor (I) ein Reaktionsgemisch enthaltend das polare Lösungsmittel, die Ameisensäure/Amin-Addukte, das tertiäre Amin und den Katalysator, bildet, das aus dem Reaktor als Austrag (3) ausgetragen wird,

dadurch gekennzeichnet, dass

der Austrag (3) aus dem Hydrierreaktor (I), nach Zugabe von Wasser, direkt einer Extraktionseinheit (II) zugeführt wird und die Aufarbeitung des Austrags (3) die folgenden Verfahrensschritte umfasst:

1) Extraktion des Katalysators aus dem Austrag (3) aus dem Hydrierreaktor (I) in der Extraktionseinheit (II), wobei als Extraktionsmittel das gleiche tertiäre Amin, das in der Hydrierung eingesetzt wurde, eingesetzt wird, unter Erhalt eines Extrakts (4), der den Hauptteil des tertiären Amins und des Katalysators enthält, und der in den Hydrierreaktor (I) recycliert wird sowie eines Raffinats (5), das den Hauptteil des polaren Lösungsmittels und der Ameisensäure/Amin-Addukte enthält, und in das eine Destillationseinheit (III) weitergeleitet wird, worin

2) eine Destillation durchgeführt wird unter Erhalt eines Kopfstroms (6), der überwiegend das polare Lösungsmittel enthält, und der in den Hydrierreaktor (I) recycliert wird, sowie eines Sumpfstroms (7), der überwiegend die Ameisensäure/Amin-Addukte und das tertiäre Amin enthält, und welcher gemäß Verfahrensschritt

3) einem Phasentrenngefäß (IV) zugeführt wird, worin

a) eine Phasentrennung durchgeführt wird unter Erhalt einer Oberphase, enthaltend überwiegend das tertiäre Amin, die als Strom (11) in die Extraktionseinheit (II) als Extraktionsmittel für den Katalysator recycliert wird, und einer Unterphase, enthaltend überwiegend die Ameisensäure/Amin-Addukte, die als Strom (8) einer thermischen Spalteinheit (V) zugeführt wird, worin

b) eine thermische Spaltung durchgeführt wird unter Erhalt eines das tertiäre Amin enthaltenden Stroms (9), der in das Phasentrenngefäß (IV) zurückgeführt wird und eines die Ameisensäure enthaltenden Stroms (10), oder gemäß Verfahrensschritt

4) einer thermischen Spalteinheit (V) zugeführt wird, worin

a) eine thermische Spaltung durchgeführt wird unter Erhalt eines die Ameisensäure enthaltenden Stroms (10) und eines das tertiäre Amin und die Ameisensäure/Amin-Addukte enthaltenden Stroms (9), der einem Phasentrenngefäß (IV) zugeführt wird, worin

b) eine Phasentrennung durchgeführt wird unter Erhalt einer Oberphase, enthaltend überwiegend das tertiäre Amin, die als Strom (11) in die Extraktionseinheit (II) als Extraktionsmittel für den Katalysator recycliert wird, und einer Unterphase, enthaltend überwiegend die Ameisensäure/Amin-Addukte, die als Strom (8) einer thermischen Spalteinheit (V) zugeführt wird.

Die Zugabe von Wasser nach erfolgter Hydrierung hat die Auswirkung, dass sich die Verteilungskoeffizienten des Katalysators durch die Wasserzugabe zugunsten einer Anreicherung desselben in der Amin-Phase verbessern und dadurch eine effiziente Rückführung des Katalysator ermöglicht wird, ohne dessen Hydrieraktivität zu erniedrigen. Der Hydrieraustrag kann, je nach verwendetem polarem Lösungsmittel und Konzentration an Ameisensäure-Amin-Addukten ein- oder zweiphasig sein, wobei sich dann durch Zugabe von Wasser die Ameisensäure-Amin-Addukte in der Produktphase (Raffinat) anreichern. Es wird bevorzugt so viel Wasser zugegeben, dass ein Wassergehalt im Raffinat (5) von 0,1 bis 50 Gew.-%, bezogen auf das Gesamtgewicht des Raffinats (5) aus der Extraktionseinheit (II), erreicht wird, besonders bevorzugt 2 bis 30 Gew.-%, bezogen auf das Gesamtgewicht des Raffinats (5) aus der Extraktionseinheit (II). Das zuzuführende Wasser kann aus der Destillationseinheit (III) stammen, in welcher der größte Teil des polaren Lösungsmittels sowie das Wasser vom Raffinat (5) abgetrennt wird und/oder es kann sich auch um Wasser handeln, welches dem Prozess frisch zugeführt wird. Das Wasser kann, für den Fall, dass der Austrag (3) entspannt wird, sowohl nach der Entspannung des Austrages aus dem Hydrierreaktor auf Normaldruck, als auch vor der Entspannung desselben zugegeben werden.

In einer weiteren Ausführungsform der Erfindung wird dem Austrag (3) aus dem Hydrierreaktor (I) kein Wasser zugegeben. Gegenstand der Erfindung ist daher auch ein Verfahren zur Herstellung von Ameisensäure durch Umsetzung von Kohlendioxid (1) mit Wasserstoff (2) in einem Hydrierreaktor (I) in Gegenwart

- eines Katalysators, enthaltend ein Element aus der 8., 9. oder 10. Gruppe des Periodensystems,
- eines tertiären Amins, enthaltend mindestens 12 Kohlenstoffatome pro Molekül, sowie
- eines polaren Lösungsmittels, enthaltend einen oder mehrere Monoalkohole, ausgewählt aus Methanol, Ethanol, Propanolen, Butanolen und Wasser,

unter Bildung von Ameisensäure/Amin-Addukten als Intermediaten, die anschließend thermisch gespalten werden, wobei ein tertiäres Amin eingesetzt wird, das einen um mindestens 5 °C höheren Siedepunkt als Ameisensäure aufweist, und wobei

sich bei der Umsetzung im Hydrierreaktor (I) ein Reaktionsgemisch enthaltend das polare Lösungsmittel, die Ameisensäure/Amin-Addukte, das tertiäre Amin und den Katalysator, bildet, das aus dem Reaktor als Austrag (3) ausgetragen wird,

dadurch gekennzeichnet, dass

der Austrag (3) aus dem Hydrierreaktor (I) direkt einer Extraktionseinheit (II) zugeführt wird und die Aufarbeitung des Austrags (3) die folgenden Verfahrensschritte umfasst:

1) Extraktion des Katalysators aus dem Austrag (3) aus dem Hydrierreaktor (I) in der Extraktionseinheit (II), wobei als Extraktionsmittel das gleiche tertiäre Amin, das in der Hydrierung eingesetzt wurde, eingesetzt wird, unter Erhalt eines Extrakts (4), der den Hauptteil des tertiären Amins und des Katalysators enthält, und der in den Hydrierreaktor (I) recycliert wird sowie eines Raffinats (5), das den Hauptteil des polaren Lösungsmittels und der Ameisensäure/Amin-Addukte enthält, und das in eine Destillationseinheit (III) weitergeleitet wird, worin

2) eine Destillation durchgeführt wird unter Erhalt eines Kopfstroms (6), der überwiegend das polare Lösungsmittel enthält, und der in den Hydrierreaktor (I) recycliert wird, und eines Sumpfstroms (7), der überwiegend die Ameisensäure/Amin-Addukte und das tertiäre Amin enthält, und welcher gemäß Verfahrensschritt

3) einem Phasentrenngefäß (IV) zugeführt wird, worin

a) eine Phasentrennung durchgeführt wird unter Erhalt einer Oberphase, enthaltend überwiegend das tertiäre Amin, die als Strom (11) in die Extraktionseinheit (II) als Extraktionsmittel für den Katalysator recycliert wird, und einer Unterphase, enthaltend überwiegend die Ameisensäure/Amin-Addukte, die als Strom (8) einer thermischen Spalteinheit (V) zugeführt wird, worin

b) eine thermische Spaltung durchgeführt wird unter Erhalt eines das tertiäre Amin enthaltenden Stroms (9), der in das Phasentrenngefäß (IV) zurückgeführt wird und eines die Ameisensäure enthaltenden Stroms (10),

oder gemäß Verfahrensschritt

4) einer thermischen Spalteinheit (V) zugeführt wird, worin

a) eine thermische Spaltung durchgeführt wird unter Erhalt eines die Ameisensäure enthaltenden Stroms (10) und eines das tertiäre Amin und die Ameisensäure/Amin-Addukte enthaltenden Stroms (9), der einem Phasentrenngefäß (IV) zugeführt wird, worin

b) eine Phasentrennung durchgeführt wird unter Erhalt einer Oberphase, enthaltend überwiegend das tertiäre Amin, die als Strom (11) in die Extraktionseinheit (II) als Extraktionsmittel für den Katalysator recycliert wird, und einer Unterphase, enthaltend überwiegend die Ameisensäure/Amin-Addukte, die als Strom (8) einer thermischen Spalteinheit (V) zugeführt wird.

Bevorzugt werden hierbei die Verhältnisse von Monoalkohol(en), tertiärem Amin und Wasser im Hydrierreaktor (I) so gewählt, dass ein Wassergehalt im Raffinat (5) von 0,1 bis 50 Gew.-%, bezogen auf das Gesamtgewicht des Raffinats (5) aus der Extraktionseinheit (II) erreicht wird, besonders bevorzugt 2 bis 30 Gew.-%, bezogen auf das Gesamtgewicht des Raffinats (5) aus der Extraktionseinheit II.

Der in der Extraktionseinheit (II) erhaltene Extrakt (4) enthält den Hauptteil des tertiären Amins und des Katalysators. Das in der Extraktionseinheit (II) erhaltene Raffinat (5) enthält den Hauptteil des polaren Lösungsmittels und der Ameisensäure/Amin-Addukte.

"Hauptteil" in Bezug auf das Extrakt (4) bedeutet dabei, dass das Extrakt (4) eine höhere Konzentration an tertiärem Amin und Katalysator aufweist als das Raffinat (5). Unter tertiärem Amin wird dabei das Amin verstanden, das in freier Form vorliegt und nicht in Form des Ameisensäure/Aminadduktes an Ameisensäure gebunden ist.

"Hauptteil" in Bezug auf das Raffinat (5) bedeutet dabei, dass das Raffinat (5) eine höhere Konzentration an polarem Lösungsmittel und den Ameisensäure/Amin-Addukten aufweist als das Extrakt (4).

In einer Ausführungsform des erfindungsgemäßen Verfahrens wird ein Teilstrom des in der Destillationseinheit (III) erhaltenen Sumpfstroms (7) dem Phasentrenngefäß (IV) zugeführt und der Sumpfstroms (7) im Übrigen wird der thermischen Spalteinheit (V) zugeführt. Ein Teilstrom des Sumpfstroms (7) wird dabei gemäß Verfahrensschritt 3) und der Sumpfstrom im Übrigen gemäß Verfahrensschritt 4) aufgearbeitet.

Das bei der Hydrierung von Kohlendioxid einzusetzende Kohlendioxid kann fest, flüssig oder gasförmig eingesetzt werden. Es ist auch möglich, großtechnisch verfügbare, Kohlendioxid enthaltende Gasgemische zu verwenden, sofern diese weitgehend frei von Kohlenmonoxid, d.h. einen Volumenanteil von <1 % CO, sind. Der bei der Hydrierung

von Kohlendioxid einzusetzende Wasserstoff ist im Allgemeinen gasförmig. Kohlendioxid und Wasserstoff können auch inerte Gase, wie etwa Stickstoff oder Edelgase, enthalten. Vorteilhafterweise liegt jedoch deren Gehalt unterhalb von 10 mol-% bezogen auf die Gesamtmenge an Kohlendioxid und Wasserstoff im Hydrierreaktor. Größere Mengen sind zwar gegebenenfalls ebenfalls noch tolerierbar, bedingen aber im Allgemeinen die Anwendung eines höheren Druckes im Reaktor, wobei dadurch weitere Kompressionsenergie erforderlich ist und der apparative Aufwand steigt.

Die Hydrierung von Kohlendioxid erfolgt in der Flüssigphase bevorzugt bei einer Temperatur von 20 bis 200°C und einem Gesamtdruck von 0,2 bis 30 MPa abs. Bevorzugt beträgt die Temperatur mindestens 30°C und besonders bevorzugt mindestens 40°C sowie bevorzugt höchstens 150°C, besonders bevorzugt höchstens 120°C und ganz besonders bevorzugt höchstens 80°C. Der Gesamtdruck beträgt bevorzugt mindestens 1 MPa abs und besonders bevorzugt mindestens 3 MPa abs sowie bevorzugt höchstens 15 MPa abs.

Der Partialdruck des Kohlendioxids beträgt dabei im Allgemeinen mindestens 0,5 MPa und bevorzugt mindestens 1 MPa sowie im Allgemeinen höchstens 8 MPa. Der Partialdruck des Wasserstoffs beträgt im Allgemeinen mindestens 0,5 MPa und bevorzugt mindestens 1 MPa sowie im Allgemeinen höchstens 25 MPa und bevorzugt höchstens 10 MPa.

Das Molverhältnis von Wasserstoff zu Kohlendioxid im Zulauf des Hydrierreaktors beträgt bevorzugt 0,1 bis 10 und besonders bevorzugt 0,2 bis 5, insbesondere bevorzugt 0,5 bis 3.

Das Molverhältnis von Kohlendioxid zu tertiärem Amin im Zulauf des Hydrierreaktors beträgt im Allgemeinen 0,1 bis 10 und bevorzugt 0,2 bis 5, insbesondere bevorzugt 0,5 bis 3.

Als Hydrierreaktoren können prinzipiell alle Reaktoren eingesetzt werden, welche für gas/flüssig-Reaktionen unter der gegebenen Temperatur und dem gegebenen Drucks grundsätzlich geeignet sind. Geeignete Standardreaktoren für gas-flüssig und für flüssig-flüssig Reaktionssyteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren.

Die Hydrierung von Kohlendioxid kann beim erfindungsgemäßen Verfahren diskontinuierlich oder kontinuierlich durchgeführt werden. Bei der diskontinuierlichen Fahrweise wird der Reaktor mit den gewünschten flüssigen und gegebenenfalls festen Einsatz-und Hilfsstoffen bestückt und anschließend Kohlendioxid und Wasserstoff auf den gewünschten Druck bei der gewünschten Temperatur aufgepresst. Nach Ende der Reaktion wird der Reaktor im Regelfall entspannt und die beiden gebildeten Flüssigphasen voneinander getrennt. Bei der kontinuierlichen Fahrweise werden die Einsatz- und Hilfsstoffe, einschließlich des Kohlendioxids und Wasserstoffs kontinuierlich zugegeben. Entsprechend wird die Flüssigphase kontinuierlich aus dem Reaktor abgeführt, so dass der Flüssigkeitsstand im Reaktor im Mittel gleich bleibt. Bevorzugt ist die kontinuierliche Hydrierung von Kohlendioxid.

Die mittlere Verweilzeit im Hydrierreaktor beträgt im Allgemeinen 5 Minuten bis 5 Stunden.

Die bei der Hydrierung von Kohlendioxid in Gegenwart des einzusetzenden Katalysators, des polaren Lösungsmittels und des tertiären Amins gebildeten Ameisensäure/Amin-Addukte weisen im Allgemeinen die allgemeine Formel (IIa)

$$NR^1R^2R^3 \cdot x_iHCOOH \qquad (IIa),$$

auf, in der die Reste $R^1$ bis $R^3$ den beim tertiären Amin (Ia) beschriebenen Resten entsprechen und $x_1$ gleich 0.4 bis 5, bevorzugt 0.7 bis 1.6 beträgt. Die jeweilige gemittelte Zusammensetzung des Amin-Ameisensäureverhältnisses in den Produktphasen bei den jeweiligen Verfahrensschritten, d.h. der Faktor $x_i$, lassen sich beispielsweise durch Bestimmung des Ameisensäuregehaltes durch Titration mit einer alkoholischen KOH-Lösung gegen Phenolphthalein und des Amingehaltes durch Gaschromatographie bestimmen. Die Zusammensetzung der Ameisensäure/Amin-Addukte, d.h. der Faktor $x_i$ kann sich während der verschiedenen Verfahrensschritte ändern. So bilden sich zum Beispiel im Allgemeinen nach der Entfernung des polaren Lösungsmittel Addukte mit einem höheren Ameisensäuregehalt mit $x_2 > x_1$ mit $x_2$ 1 bis 4 aus, wobei das überschüssige, freie Amin eine Zweitphase ausbilden kann.

Der Austrag (3) aus dem Hydrierreaktor (I), der das polare Lösungsmittel, die Ameisensäure/Amin-Addukte, das tertiäre Amin und den Katalysator enthält, und dem gegebenenfalls Wasser zugegeben wurde, wird mit aus den entsprechenden Phasentrenngefäßen stammendem Strömen von freiem tertiärem Amin extrahiert und in den Hydrierreaktor rückgeführt. Dies geschieht, um den Katalysator abzutrennen. Ohne diese Extraktion könnte Hydrierkatalysator in die Vorrichtung zur thermischen Spaltung des Addukts aus tertiärem Amin und Ameisensäure gelangen und dort die Zersetzung von Ameisensäure katalysieren und so die Ameisensäureausbeute mindern. Restmengen aus Wasserstoff und Kohlendioxid werden als Abgas entsorgt.

Die Extraktion des Katalysators aus dem Austrag des Hydrierreaktors kann beispielsweise nach Entspannung, beispielsweise auf etwa oder nahe Atmosphärendruck, und Abkühlung des Austrags (3), beispielsweise auf etwa oder nahe Umgebungstemperatur, erfolgen.

Hierbei ist das bei der Extraktion erhaltene Extrakt (4), das das tertiäre Amin und den Katalysator enthält, vor

der Rückführung zum Hydrierreaktor (I) separat auf Reaktionsdruck zu bringen. Dabei sind für die rückzuführenden Gas- und Flüssigphasen ein oder mehrere geeignete, entsprechend der zu überwindenden Druckdifferenz ausgelegte Verdichter bzw. eine Pumpe vorzusehen.

Im Rahmen der vorliegenden Erfindung wurde überraschend gefunden, dass sich bei der Extraktion ein mit den Ameisensäure/Amin-Addukten sowie dem polaren Lösungsmittel angereichertes Raffinat (5) und ein mit dem tertiären Amin und im Falle des Einsatzes eines homogenen Katalysators auch mit diesem angereichertes Extrakt (4) auch bei einem deutlich erhöhten Druck erhalten werden kann. Daher können im erfindungsgemäßen Verfahren die Lösungsmittel so gewählt werden, dass die Trennung der einen, mit den Ameisensäure/Amin-Addukten und dem polaren Lösungsmittel angereicherte Phase von der anderen, mit dem tertiären Amin angereicherten Phase, sowie die Rückführung der mit dem tertiären Amin angereicherten Phase zum Hydrierreaktor bei einem Druck von 0,1 bis 30 MPa abs stattfinden kann. Gemäß dem Gesamtdruck im Hydrierreaktor, liegt der Druck bei bevorzugt höchstens 15 MPa abs. So ist es sogar möglich, ohne vorhergehende Entspannung beide Flüssigphasen voneinander zu trennen und das Extrakt (4) ohne nennenswerte Druckerhöhung aus der Extraktion zum Hydrierreaktor zurückzuführen. In diesem Falle, sowie auch im Falle einer nur geringfügigen Entspannung ist es möglich, auf eine Rückführung einer etwaigen Gasphase gänzlich zu verzichten.

Das erfindungsgemäße Verfahren kann in einer Ausführungsform somit in der Weise durchgeführt werden, dass der Druck und die Temperatur im Hydrierreaktor und in der Extraktionseinheit (II) gleich oder annähernd gleich sind, wobei unter annähernd gleich vorliegend ein Druckunterschied von bis zu +/- 5 bar bzw. ein Temperaturunterschied von bis zu +/- 5 °C verstanden wird.

Überraschenderweise wurde auch gefunden, dass sich bei dem vorliegenden System beide Flüssigphasen auch bei einer erhöhten Temperatur, die der Reaktionstemperatur entspricht, sehr gut voneinander trennen können. Insofern ist zur Phasentrennung auch keine Abkühlung und anschließende Aufheizung der rückzuführenden Oberphase erforderlich, was ebenfalls Energie einspart.

Die Extraktion wird bevorzugt bei Temperaturen im Bereich von 20 bis 100 °C, besonders bevorzugt bei 40 bis 80 °C durchgeführt.

Die Extraktion des Hydrierkatalysators kann in jeder geeigneten, dem Fachmann bekannten Vorrichtung durchgeführt werden, bevorzugt in Gegenstrom-Extraktionskolonnen, Mixer-Settler-Kaskaden oder Kombinationen von Mixer-Settlern mit Kolonnen.

Das Raffinat (5) aus der Extraktionseinheit (II), das den Hauptteil des polaren Lösungsmittels und der Ameisensäure/Amin-Addukte enthält, wird der Destillationseinheit (III) zugeführt, um das polare Lösungsmittel oder -gemisch vom Ameisensäure/Amin-Addukt zu trennen. Die aus der Extraktionseinheit (II) tertiäres Amin und Hydrierkatalysator enthaltende Phase wird in den Hydrierreaktor zurückgeführt.

Unter Umständen werden neben dem Katalysator Anteile einzelner Komponenten des polaren Lösungsmittels aus der zu extrahierenden Flüssigphase im Extraktionsmittel, dem Aminstrom, gelöst. Dies ist für das Verfahren kein Nachteil, da die bereits extrahierte Menge an Lösungsmittel nicht der Lösungsmittelabtrennung zugeführt werden muss und somit unter Umständen Verdampfungsenergie und Apparatekosten einspart.

Es kann vorteilhaft sein, zwischen der Extraktionseinheit (II) und der Destillationseinheit (III) eine Vorrichtung zur Adsorption von Spuren von Hydrierkatalysator zu integrieren. Zahlreiche Adsorptionsmittel sind für die Adsorption geeignet. Beispiele hierfür sind Polyacrylsäure und Salze dieser, sulfonierte Polystyrole und Salze dieser, Aktivkohlen, Montmorillonite, Bentonite, Kieselgele sowie Zeolithe.

Bei der Extraktion des Katalysators aus Austrag (3) in der Extaktionseinheit (II) wird ein Extrakt (4), das das tertiäre Amin und den Katalysator enthält, und ein Raffinat (5), das das polare Lösungsmittel und die Ameisensäure/Amin-Addukte enthält, erhalten. Das Raffinat (5) ist mit den Ameisensäure/Amin-Addukten sowie dem polaren Lösungsmittel angereichert. Hinsichtlich der Ameisensäure/Amin-Addukte ist unter "angereichert" ein Verteilungskoeffizient der Ameisensäure/Amin-Addukte

$$P = [\text{Konzentration Ameisensäure/Amin-Addukt (II) in Flüssigphase (A)}] / [\text{Konzentration Ameisensäure/Amin-Addukt (II) in Flüssigphase (B)}]$$

von > 1 zu verstehen. Bevorzugt liegt der Verteilungskoeffizient bei ≥ 2 und besonders bevorzugt bei ≥ 5. Das Extrakt (4) ist mit dem tertiären Amin angereichert. Im Falle des Einsatzes eines homogenen Katalysators liegt dieser ebenfalls im Raffinat angereichert vor. Das Extrakt (4) wird in den Hydrierreaktor recycliert.

Die Flüssigphasen (A) und (B) haben dabei die bereits vorstehend definierte Bedeutung.

Gegebenenfalls ist auch eine Rückführung einer Gasphase enthaltend nicht umgesetztes Kohlendioxid und/oder nicht umgesetzten Wasserstoff zum Hydrierreaktor vorteilhaft. Gegebenenfalls ist es beispielsweise zur Ausschleusung von unerwünschten Nebenprodukten oder Verunreinigungen wünschenswert, einen Teil der Oberphase und/oder einen Teil der Kohlendioxid oder Kohlendioxid und Wasserstoff enthaltenden flüssigen beziehungsweise gasförmigen Phasen aus dem Verfahren auszuschleusen. Der Großteil des polaren Lösungsmittels des Raffinats (5) wird in einer Destillationseinheit (III) thermisch von den Ameisensäure/Amin-Addukten abgetrennt, wobei das destillativ entfernte polare Lösungsmittel zum Hydrierreaktor (I) rückgeführt wird. Im Sumpf der Destillationseinheit (III) fallen die reinen Ameisensäure/Amin-Addukte sowie freies tertiäres Amin an, da sich bei der Entfernung des polaren Lösungsmittels Ameisensäure/Amin-Addukte mit niedrigerem Amingehalt bilden, wodurch sich ein zweiphasiges Sumpfgemisch bestehend aus einer Amin- und einer Ameisensäure/Amin-Addukt-Phase bildet.

Die thermische Trennung des polaren Lösungsmittels oder -gemisches, siehe oben, erfolgt bevorzugtermaßen bei einer Sumpftemperatur, bei welcher bei gegebenem Druck sich keine freie Ameisensäure aus dem Ameisensäure/Amin-Addukt mit dem höheren (x1) oder niedrigerem (x2) Amingehalt bildet. Im Allgemeinen beträgt die Sumpftemperatur der thermischen Abtrenneinheit mindestens 20°C, bevorzugtermaßen mindestens 50°C und besonders bevorzugtermaßen mindestens 70°C sowie im Allgemeinen höchstens 210°C, bevorzugtermaßen höchstens 190°C. Der Druck beträgt dabei im Allgemeinen mindestens 1 hPa abs, bevorzugt mindestens 50 hPa abs und besonders bevorzugt mindestens 100 hPa abs sowie im Allgemeinen höchstens 1 MPa abs und bevorzugtermaßen 0,1 MPa abs.

Die thermische Abtrennung des polaren Lösungsmittels oder -gemisches erfolgt entweder in einem Verdampfer oder in einer Destillationseinheit, bestehend aus Verdampfer und Kolonne, gefüllt mit Packung, Füllkörpern und/oder Böden. Das Lösungsmittel kann nach der thermischen Trennung kondensiert werden, wobei wiederum die dabei freiwerdende Kondensationsenthalpie genutzt werden kann, um beispielsweise das aus der Extraktion kommende Lösungsmittel mit Amin/Ameisensäureaddukt-Gemisch auf Abdampftemperatur vorzuwärmen.

Alternativ können auch nur Teile des Lösungsmittelgemisches abgetrennt werden. Dies gilt insbesondere für Lösungsmittelkomponenten, die in der späteren Ameisensäuredestillation über einen Seitenstrom abgetrennt werden können.

Die Ameisensäure/Amin-Addukte, die nach der thermischen Abtrennung des polaren Lösungsmittels oder -gemisches oder Teilen des Lösungsmittels in der Destillationseinheit (III) anfallen, werden dann als Sumpfstrom (7) in einer thermischen Spalteinheit (V), beispielsweise einer Destillationseinheit, thermisch in freie Ameisensäure und freies tertiäres Amin gespalteten, wobei die gebildete freie Ameisensäure destillativ entfernt und das im Sumpf der Destillationseinheit enthaltene freie tertiäre Amin zum Hydrierreaktor (I) rückgeführt wird. Dabei kann das bei der thermischen Abtrennung des polaren Lösungsmittels in der Destillationseinheit (III) als Zweitphase anfallende freie Amin zuvor in einem Phasentrenngefäß abgetrennt werden, in einem gemeinsamen Phasentrenngefäß zusammen mit dem Sumpfprodukt der thermischen Spalteinheit (V) zur Ameisensäureabtrennung oder als zweiphasiges Gemisch direkt der Spalteinheit (V) zugeführt werden (siehe allgemeine Ausführungsformen). Die Entnahme der freigesetzten Ameisensäure kann dabei beispielsweise (i) über Kopf, (ii) über Kopf und als Seitenabzug oder (iii) nur als Seitenabzug erfolgen. Wird Ameisensäure über Kopf entnommen, so ist dabei eine Ameisensäure-Reinheit von bis zu 99,99 Gew.-% möglich. Bei einer Entnahme als Seitenabzug wird wässrige Ameisensäure erhalten, wobei hierbei das Gemisch mit etwa 85 Gew.-% Ameisensäure in der Praxis von besonderer Bedeutung ist. Je nach dem Wassergehalt des Zulaufs zur Destillationseinheit wird die Ameisensäure verstärkt als Kopfprodukt oder als Seitenprodukt entnommen. Bei Bedarf ist es sogar möglich, Ameisensäure nur als Seitenprodukt zu entnehmen, wobei dann gegebenenfalls die erforderliche Wassermenge sogar noch explizit zugegeben werden kann. Die thermische Spaltung des Ameisensäure/Amin-Addukts erfolgt im Allgemeinen nach den im Stand der Technik bekannten Verfahrensparametern hinsichtlich Druck, Temperatur sowie apparativer Ausgestaltung. So sei beispielsweise auf die Beschreibungen in EP 0 181 078 A oder WO 2006/021,411 verwiesen. Die einzusetzende Destillationseinheit umfasst in der Regel eine Destillationskolonne, welche im Allgemeinen Füllkörper, Packungen und/oder Böden enthält.

Im Allgemeinen beträgt die Sumpftemperatur in der Destillationskolonne (Spalteinheit (V)) mindestens 130°C, bevorzugt mindestens 140°C und besonders bevorzugt mindestens 150°C, sowie im Allgemeinen höchstens 210°C, bevorzugt höchstens 190°C und besonders bevorzugt höchstens 185°C. Der Druck beträgt im Allgemeinen mindestens 1 hPa abs, bevorzugt mindestens 50 hPa abs und besonders bevorzugt mindestens 100 hPa abs, sowie im Allgemeinen höchstens 500 hPa abs, bevorzugt höchstens 300 hPa abs und besonders bevorzugt höchstens 250 hPa abs.

Gegebenenfalls wird als Seitenprodukt noch ein wasserhaltiger Strom an Ameisensäure entnommen.

Das erfindungsgemäße Verfahren besitzt gegenüber den in EP 181.078 B1 und EP 357.243 B1 vorbeschriebenen integrierten Verfahren eine Reihe von Vorteilen: Für das Abfangen der Ameisensäure in der Hydrierung und die thermische Spaltung der Ameisensäure/Amin-Addukte wird das gleiche tertiäre Amin verwendet. Dieses Amin, das bei der thermischen Spaltung frei anfällt, wird dann zur Extraktion des Katalysators in der Produktphase eingesetzt, um den Katalysator mit dem Amin in das Reaktionsgefäß zurückzuführen. Es besitzt eine höhere Stabilität als die vorbeschriebenen N-Alkylimidazole. Verluste an Edelmetallen treten praktisch nicht auf. Es wird verhindert, dass der Katalysator in die thermische Spalteinheit gelangt und darin die Zersetzung von Ameisensäure katalysiert. Von großem Vorteil ist, dass er in seiner aktiven Form abgetrennt und zurückgeführt werden kann. Es werden hohe Ameisensäure-Ausbeuten und eine hohe Produktreinheit erzielt. An die Stelle von zwei Destillationen tritt die Extraktion. Hierdurch werden Energie- und Investitionskosten gesenkt.

Das erfindungsgemäße Verfahren ermöglicht die Gewinnung von konzentrierter Ameisensäure in hoher Ausbeute und hoher Reinheit durch Hydrierung von Kohlendioxid. Es zeichnet sich insbesondere durch eine besonders einfache Verfahrensführung aus, welche gegenüber dem Stand der Technik ein einfacheres Verfahrenskonzept, einfachere Verfahrensstufen, eine geringere Anzahl an Verfahrensstufen sowie einfachere Apparate aufweist. So wird beispielsweise durch geeignete Wahl des tertiären Amins und des polaren Lösungsmittels im Falle des Einsatzes eines homogenen Katalysators dieser durch Extraktion von Ameisensäure/Amin-Addukten getrennt und ohne weitere Aufarbeitungsschritte zum Hydrierreaktor rückgeführt. Die Extraktion kann dabei auch unter Druck erfolgen. Aufgrund der zügigen Abtrennung des Katalysators von den gebildeten Ameisensäure/Amin-Addukten wird eine Rückreaktion unter Zersetzung in Kohlendioxid und Wasserstoff unterdrückt.

Ferner ist beim erfindungsgemäßen Verfahren kein aufwändiger, separater Basenaustausch erforderlich, so dass die im Hydrierreaktor gebildeten Ameisensäure/Amin-Addukte direkt zur thermischen Spaltung eingesetzt werden können. Der Einsatz eines niedrigsiedenden polaren Lösungsmittels ermöglicht dessen thermische Abtrennung in einer der thermischen Spaltung der Ameisensäure vorgelagerten Stufe unter milden Bedingungen, wodurch die Veresterung eingesetzter Alkohole und die Zersetzung der Ameisensäure minimiert wird, ein geringerer Energiebedarf nötig ist und eine höhere Reinheit der Ameisensäure erzielt werden kann. Als Folge des einfacheren Verfahrenskonzepts ist die zur Durchführung des erfindungsgemäßen Verfahrens erforderliche Produktionsanlage gegenüber dem Stand der Technik kompakter im Sinne eines geringeren Platzbedarfs und des Einsatz von wenigeren Apparaten. Sie weist einen geringeren Investitionsaufwand und einen geringeren Energiebedarf auf.

Die Erfindung wird im Folgenden anhand von Ausführungsbeispielen sowie einer Zeichnung näher erläutert.

Beispiel A1-4 Hydrierung, Katalysatorextraktion und Wiederverwendung des Katalysators:

Ein 100 mL bzw. 250 mL Autoklav aus Hastelloy C, ausgestattet mit einem Blatt- oder Magnetrührer, wurde unter Inertbedingungen mit dem Trialkylamin, polaren Lösungsmittel und dem Katalysator befüllt. Anschließend wurde der Autoklav verschlossen und bei Raumtemperatur wurde $CO_2$ aufgepresst. Nachfolgend wurde $H_2$ aufgepresst und der Reaktor unter Rühren (700-1000 U/min) erwärmt. Nach der Reaktionszeit wurde der Autoklav abgekühlt und das Reaktionsgemisch entspannt. Nach der Reaktion wurde zum Reaktionsaustrag Trialkylamin und ggf. Wasser zur Extraktion zugegeben und 10 Minuten bei Raumtemperatur gerührt. Es wurde ein zweiphasiges Produktgemisch erhalten, wobei die obere Phase mit dem tertiären Amin und dem homogenen Katalysator, und die untere Phase mit dem polaren Lösungsmittel und dem gebildeten Ameisensäure/Amin-Addukt angereichert war. Die Phasen wurden anschließend getrennt der Ameisensäuregehalt der Unterphase bestimmt. Die Oberphase, enthaltend Rutheniumkatalysator und das freier Amin, wurde dann mit dem gleichen Lösungsmittel unter den gleichen Reaktionsbedingungen wie zuvor erneut zur $CO_2$-Hydrierung eingesetzt. Nach vollendeter Reaktion wurde frisches Trialkylamin zugegeben um den Katalysator zu extrahieren. Die Produktphase wurde anschließend abgetrennt und der Ameisensäuregehalt sowie der Rutheniumgehalt beider Phasen bestimmt. Der Gesamtgehalt an Ameisensäure im Ameisensäure/Amin-Addukt wurde durch Titration mit 0,1 N KOH in MeOH potentiometrisch mit einem "Mettler Toledo DL50"-Titrator bestimmt. Der Rutheniumgehalt wurde per AAS-bestimmt. Die Parameter und Ergebnisse der einzelnen Versuche sind in den Tabellen 1.1 bis 2.2 wiedergegeben.

Die Beispiele A-1 bis A-4 zeigen, dass sich der aktive Katalysator durch Extraktion von der Produktphase abtrennen und für die Hydrierung wiederverwenden lässt. Höhere Abreicherungsraten für das Ruthenium können durch einfaches Hintereinanderschalten mehrerer Extraktionsschritte wie z.B. in einer Mixer-Settler-Kaskade oder in einer Gegenstromextraktion mit der gleichen Menge Amin erreicht werden.

**Tabelle 1.1**

| | Beispiel A-1a (erste Hydrierung und Extraktion) | Beispiel A-1b (Wiederverwendung des Katalysators und Extraktion) | Beispiel A-2a (erste Hydrierung) | Beispiel A-2b (Wiederverwendung des Katalysators und Extraktion) |
|---|---|---|---|---|
| Autoklav | 250 mL | 250 mL | 100 mL | 100 mL |
| Tertiäres Amin | 65.0 g Trihexylamin | Oberphase aus A-1a | 20.0 g Trihexylamin | 15.0 g Oberphase aus A-2a |
| Polares Lösungsmittel (eingesetzt) | 25.0 g Methanol<br>2.0 g Wasser | 25.0 g Methanol<br>2.0 g Wasser | 10.0 g Methanol<br>1.0 g Wasser | 10.0 g Methanol<br>1.0 g Wasser |
| Katalysator | 0.32 g $[Ru(PnOctyl_3)_4(H)_2]$, 0.08 g 1,2-Bis(dicyclohexylphosphino)ethan | Oberphase aus A-1a | 0.16 g $[Ru(PnOctyl_3)_4(H)_2]$, 0.04 g 1,2-Bis(dicyclohexylphosphino)ethan | 15.0 g Oberphase aus A-2 |
| Aufpressen von $CO_2$ | Auf 3.1 MPa abs | Auf 3.0 MPa abs | Auf 2.2 MPa abs | Auf 2.4 MPa abs. |
| Aufpressen von $H_2$ | Auf 12.0 MPa abs | Auf 12.0 MPa | Auf 8.0 MPa abs | Auf 8.0 MPa abs |
| Erwärmen | 70°C | 70°C | 70°C | 70°C |
| Reaktionszeit | 1 Stunde | 1 Stunde | 1 Stunde | 1 Stunde |
| Aminzugabe nach der Reaktion zur Extraktion | 65.0 g Trihexylamin | 65.0 g Trihexylamin | 15.0 g Trihexylamin | 15.1 g Trihexylamin |
| Wasserzugabe nach der Reaktion | - | 4.0 g | - | - |
| Oberphase | 77.3 g | 118.0 g | 19,6 g | 25.8 g |
| Unterphase | 80.9 g<br>8.6 % Ameisensäure | 50.5 g<br>6.4 % Ameisensäure | 26.0 g<br>7.73 % Ameisensäure | 17.6 g<br>5.3 % Ameisensäure |
| $C_{Ru}$ Trialkylaminphase nach Extraktion | - | 210 ppm | 290 ppm | 170 ppm |
| $C_{Ru}$ Unterphase nach Extraktion | - | 3 ppm | 75 ppm | 18 ppm |

**Tabelle 1.2**

| | Beispiel A-3a (erste Hydrierung und Extraktion) | Beispiel A-3b (Wiederver-wendung des Katalysators und Extraktion) | Beispiel A-4a (erste Hydrierung) | Beispiel A-4b (Wiederverwendung des Katalysators und Extraktion) |
|---|---|---|---|---|
| Autoklav | 250 mL | 250 mL | 250 mL | 250 mL |
| Tertiäres Amin | 65.0 g Trihexylamin | Oberphase aus A-3a | 65.0 g Trihexylamin | Oberphase aus A-4a |
| Polares Lösungsmittel (eingesetzt) | 25.0 g Methanol | 25.0 g Methanol | 25.0 g Methanol<br>3.0 g Wasser | 25.0 g Methanol |
| Katalysator | 0.16 g $[Ru(P\textit{n}Octyl_3)_4(H)_2]$, 0.04 g 1,2-Bis(dicyclohexylphosphino)ethan | Oberphase aus A-3a | 0.16 g $[Ru(P\textit{n}Octyl_3)_4(H)_2]$, 0.04 g 1,2-Bis(dicyclohexylphosphino)ethan | Oberphase aus A-4a |
| Aufpressen von $CO_2$ | Auf 2.4 MPa abs | Auf 2.3 MPa abs | Auf 2.7MPa abs | Auf 2.2 MPa abs |
| Aufpressen von $H_2$ | Auf 12.0 MPa abs | Auf 12.0 MPa | Auf 12.0 MPa abs | Auf 12.0 MPa |
| Erwärmen | 70°C | 70°C | 70°C | 70°C |
| Reaktionszeit | 1 Stunde | 1 Stunde | 1 Stunde | 1 Stunde |
| Aminzugabe nach der Reaktion zur Extraktion | 66.0 g Trihexylamin | - | 66.6 g Trihexylamin | - |
| Wasserzugabe nach der Reaktion | 4.1 g | 4.0 g | - | - |
| Oberphase | 97.7 g | 67.3 g | 91.5 g | 67.0 g |
| Unterphase | 61.7 g<br>8.3 % Ameisensäure | 54.6 g<br>6.0 % Ameisensäure | 69.1 g<br>8.6 % Ameisensäure | 50.0 g<br>5.0 % Ameisensäure |
| $C_{Ru}$ Trialkylaminphase nach Extraktion | 75 ppm | 95 ppm | 80 ppm | 100 ppm |
| $C_{Ru}$ Unterphase nach Extraktion | 27 ppm | 4 ppm | 25 ppm | 4ppm |

Beispiele B1 und B2 (Thermische Abtrennung des polaren Lösungsmittels von den Trialkylamin-Solvent-Ameisensäuregemischen und Spaltung des Ameisensäure-Amin-Adduktes; Verfahrenschritte 3 und 4):

Alkohol und Wasser werden von der Produktphase (enthält das Ameisensäure/Amin-Addukt) unter vermindertem Druck mittels eines Rotationsverdampfers abdestilliert. Im Sumpf entsteht ein zweiphasiges Gemisch (Trialkylamin- und Ameisensäure/Amin-Addukt-Phase) und die beiden Phasen werden getrennt. Die Zusammensetzung des Destillates (enthaltend den Großteil des Methanols sowie des Wassers), der Oberphase (enthaltend das freie Trialkylamin) und der Unterphase (enthaltend das Ameisensäure-Amin-Addukt) wurde durch Gaschromatographie und durch Titration der Ameisensäure gegen 0,1 N KOH in MeOH potentiometrisch mit einem "Mettler Toledo DL50"-Titrator bestimmt. Die Ameisensäure wird dann aus der Unterphase aus dem ersten Schritt in einer Vakuumdestillationsapparatur über eine 10 cm Vigreuxkolonne thermisch vom Trialkylamin abgespalten. Dabei wird nach vollständiger Abspaltung der Ameisensäure ein einphasiger Sumpf, bestehend aus dem reinen Trialkylamin erhalten, welches zur Extraktion des Katalysators und Rückführung in die Hydrierung eingesetzt werden kann. Im Destillat findet sich die Ameisensäure sowie Restwasser. Die Zusammensetzung des Sumpfes sowie des Destillates wurde durch Gaschromatographie und durch Titration der Ameisensäure mit gegen 0,1 N KOH in MeOH potentiometrisch mit einem "Mettler Toledo DL50"-Titrator bestimmt. Die Parameter und Ergebnisse der einzelnen Versuche sind in Tabelle 1.3 wiedergegeben.

Die Beispiele B-1 und B2 zeigen, dass sich beim erfindungsgemäßen Verfahren verschiedene polare Lösungsmittel unter milden Bedingungen von der Produktphase abtrennen lassen, wobei eine ameisensäurereichere Unterphase sowie eine Oberphase, bestehend überwiegend aus tertiärem Amin ausbildet. Von dieser ameisensäurereichen Unterphase kann dann die Ameisensäure bei höheren Temperaturen vom Trialkylamin abgespalten werden, wobei das freie Trialklyamin anfällt. Die so gewonnene Ameisensäure enthält noch etwas Wasser, welches aber durch eine Kolonne mit größerer Trennleistung von der Ameisensäure abgetrennt werden kann. Das sowohl bei der Abtrennung des Lösungsmittels als auch bei der thermischen Spaltung anfallende Trialkylamin kann dazu verwendet werden, den Katalysator im Schritt 2 aus dem Produktstrom abzureichern.

**Tabelle 1.3**

| | Beispiel B-1a (Abtrennung des polaren Solvents) | Beispiel B-1b (Spaltung des Ameisensäure-Amin-Adduktes) | Beispiel B-2a (Abtrennung des polaren Solvents | Beispiel B-2b (Spaltung des Ameisensäure-Amin-Adduktes) |
|---|---|---|---|---|
| Einsatzgemisch (Gew.%) | 199.8 g<br>8.9 % Ameisensäure<br>28.4 % Methanol<br>5.6 % Wasser<br>57.1 % Trihexylamin | Unterphase aus B1-a | 199.8 g<br>7.8 % Ameisensäure<br>33.0 % Methanol<br>15.1 % Wasser<br>44.0 % Trihexylamin | Unterphase aus B2-a |
| Ameisensäure : Amin Einsatzgemisch | 1 : 1.1 | 1 : 0.64 | 1 : 1 | 1 : 0.89 |
| Druck | 200 mbar | 90 mbar | 200 mbar | 90 mbar |
| Temperatur | 120°C | 153°C | 120°C | 153°C |
| Unterphase im Sumpf nach Destillation (Gew.%) | 79.8 g<br>22.1 % Ameisensäure<br>1.5 % Wasser<br>76.4 % Trihexylamin | 63.6 g<br>100 % Trihexylamin | 69.4 g<br>14.9 % Ameisensäure<br>6.9 % Wasser<br>78.2 % Trihexylamin | 55.5 g<br>99.7 % Trihexylamin<br>0.3 % Wasser |
| Oberphase im Sumpf nach Destillation | 50.5 g<br>100 % Trihexylamin | einphasig | 32.7 g<br>99.7 % Trihexylamin<br>0.3 % Wasser | einphasig |

(fortgesetzt)

| | Beispiel B-1a (Abtrennung des polaren Solvents) | Beispiel B-1b (Spaltung des Ameisensäure-Amin-Adduktes) | Beispiel B-2a (Abtrennung des polaren Solvents | Beispiel B-2b (Spaltung des Ameisensäure-Amin-Adduktes) |
|---|---|---|---|---|
| Destillat | 66.6 g<br>0.3 % Ameisensäure<br>81.2 % Methanol<br>18.5 % Wasser | 14.9 g<br>92.1 % Ameisensäure<br>7.9 % Wasser | 93.1 g<br>70.1 % Methanol<br>29.9 % Wasser | 12.9 g<br>85.0 % Ameisensäure<br>15 % Wasser |

Die Zeichnungen zeigen im Einzelnen:

| | |
|---|---|
| Figur 1 | ein Blockdiagramm einer bevorzugten Ausführungsform des erfindungsgemäßen Verfahrens, |
| Figur 2 | ein Blockdiagramm einer bevorzugten Ausführungsform des erfindungsgemäßen Verfahrens, |
| Figur 3 | ein Blockdiagramm einer weiteren bevorzugten Ausführungsform des erfindungsgemäßen Verfahrens. |
| Figur 4 | ein Blockdiagramm einer bevorzugten Ausführungsform des erfindungsgemäßen Verfahrens, |
| Figuren 5A, 5B und 5C | jeweils unterschiedliche, bevorzugte Varianten für die fraktionierte Abtrennung von polarem Lösungsmittel und Wasser für die bevorzugte Ausführungsform nach Figur 3. |

Bei der Ausführungsform gemäß Figur 1 werden Kohlendioxid, Strom (1), und Wasserstoff, Strom (2), in den Hydrierreaktor I geführt. Darin werden diese in Gegenwart eines Katalysators enthaltend ein Element aus der 8., 9. oder 10. Gruppe des Periodensystems, eines tertiären Amins und eines polaren Lösungsmittels zu Ameisensäure-Amin-Addukten umgesetzt. Der Austrag aus dem Hydrierreaktor (I) (Strom (3)), der ein- oder zweiphasig sein kann, wird der Extraktionseinheit II zugeführt, worin der Katalysator mit dem tertiären Amin aus dem Phasentrenngefäß (IV) (Strom (11)) extrahiert wird. Das tertiäre Amin mit dem Katalysator (Strom (6)) aus der Extraktionseinheit (II) wird in den Hydrierreaktor (I) zurückgeführt. Die Produktphase (5) aus der Extraktionseinheit (II) wird der thermischen Abtrenneinheit (III) zugeführt, um das polare Lösungsmittel sowie das Wasser thermisch von den Ameisensäure-Amin-Addukten abzutrennen. Der Strom (5) kann dabei noch zuvor über ein Adsorberbett geführt werden, um letzte Katalysatorspuren aus diesem Strom zu entfernen. Das polare Lösungsmittel, das in der Destillationseinheit III thermisch abgetrennt wird, wird in den Hydrierreaktor (I), als Strom (6), zurückgeführt und das zweiphasige Sumpfgemisch (Strom 7) aus der Destillationseinheit (III), enthaltend die Ameisensäure-Amin-Addukte und das tertiäre Amin, wird dem Phasentrenngefäß (IV) zugeführt. Die Ameisensäure-Amin-Addukte werden im Phasentrenngefäß (IV) abgetrennt und der Destillationseinheit (V) als Strom (8) zugeführt, worin diese in freie Ameisensäure und tertiäres Amin thermisch gespalten werden. Die freie Ameisensäure wird als Kopfprodukt, Strom (10), abgezogen und der zweiphasige Sumpf aus der Destillationseinheit (V), enthaltend tertiäres Amin und nicht gespaltene Ameisensäure/Amin-Addukte, Strom (9), wird erneut dem Phasentrenngefäß (IV) zugeführt. Das tertiäre Amin, welches im Phasentrenngefäß (IV) abgetrennt wird, wird als Strom (11) der Extraktionseinheit (II) zugeführt, um den Katalysator zu extrahieren bzw. Teile von Strom (11) können direkt in den Hydrierreaktor (I) zurückgeführt werden, falls nicht alles Trialkylamin zur Extraktion benötigt wird.

Bei der Ausführungsform gemäß Figur 2 werden Kohlendioxid, Strom (1), und Wasserstoff, Strom (2), in den Hydrierreaktor (I) geführt. Darin werden diese in Gegenwart eines Katalysators enthaltend ein Element aus der 8., 9. oder 10. Gruppe des Periodensystems, eines tertiären Amins und eines polaren Lösungsmittels zu Ameisensäure-Amin-Addukten umgesetzt. Der Austrag aus dem Hydrierreaktor (I) (Strom (3)), der ein- oder zweiphasig sein kann, wird der Extraktionseinheit II zugeführt, worin der Katalysator mit dem tertiären Amin aus dem Phasentrenngefäß (IV) (Strom (11)) extrahiert wird. Das tertiäre Amin mit dem Katalysator (Strom (6)) aus der Extraktionseinheit (II) wird in den Hydrierreaktor (I) zurückgeführt. Die Produktphase (5) aus der Extraktionseinheit (II) wird der thermischen Abtrenneinheit (III) zugeführt, um das polare Lösungsmittel sowie das Wasser thermisch von den Ameisensäure-Amin-Addukten abzutrennen. Der Strom (5) kann dabei noch zuvor über ein Adsorberbett geführt werden, um letzte Katalysatorspuren aus diesem Strom zu entfernen. Das polare Lösungsmittel, das in der Destillationseinheit III thermisch abgetrennt wird, wird in den Hydrierreaktor (I), als Strom (6), zurückgeführt und das zweiphasige Sumpfgemisch aus der Destillationseinheit (III), enthaltend die Ameisensäure-Amin-Addukte und das tertiäre Amin(Strom 7), Destillationseinheit (V) zugeführt. In dieser werden die Ameisensäure/Amin-Addukte in freie Ameisensäure und tertiäres Amin thermisch gespalten.

Die freie Ameisensäure wird beispielsweise als Kopfprodukt, Strom (10), entfernt. Der zweiphasige Sumpf der Destillationseinheit (V), Strom (9), bestehend aus tertiärem Amin und nicht gespaltenen Ameisensäure/Amin-Addukten, wird dem Phasentrenngefäß (IV) zugeführt. Das tertiäre Amin, welches im Phasentrenngefäß (IV) abgetrennt wird, wird der Extraktionseinheit (II) als Strom (11) zugeführt. Ein Teil von Strom (11) kann auch direkt in den Hydrierreaktor (I) zurückgeführt werden, falls nicht alles Amin für die Extraktion benötigt wird.

Bei der Ausführungsform gemäß Figur 3 werden Kohlendioxid, Strom (1), und Wasserstoff, Strom (2), in den Hydrierreaktor (I) geführt. Darin werden diese in Gegenwart eines Katalysators enthaltend ein Element aus der 8., 9. oder 10. Gruppe des Periodensystems, eines tertiären Amins und eines polaren Lösungsmittels zu Ameisensäure-Amin-Addukten umgesetzt. Der Austrag aus dem Hydrierreaktor (I) (Strom (3)), der ein- oder zweiphasig sein kann, wird mit dem überwiegend Wasser enthaltenden Strom (12) aus dem Destillationsgefäß (III) versetzt, um eine bessere Phasentrennung und Katalysatorverteilung in der Extraktion bei hoher Hydrieraktivität zu erhalten. Dieser Strom wird der Extraktionseinheit (II) zugeführt, worin der Katalysator mit dem tertiären Amin aus dem Phasentrenngefäß (IV) (Strom (11)) extrahiert wird. Das tertiäre Amin mit dem Katalysator (Strom (6)) aus der Extraktionseinheit (II) wird in den Hydrierreaktor (I) zurückgeführt. Die Produktphase (5) aus der Extraktionseinheit (II) wird der thermischen Abtrenneinheit (III) zugeführt, um das polare Lösungsmittel sowie das Wasser thermisch von den Ameisensäure-Amin-Addukten abzutrennen. Der Strom (5) kann dabei noch zuvor über ein Adsorberbett geführt werden, um letzte Katalysatorspuren aus diesem Strom zu entfernen Das polare Lösungsmittel, das in der Destillationseinheit III thermisch abgetrennt wird, wird in den Hydrierreaktor I, als Strom (6), zurückgeführt, das Wasser wird als Strom (12) zum Austrag aus dem Hydrierreaktor (Strom (3)) zurückgeführt, und das zweiphasige Sumpfgemisch aus der Destillationseinheit III, enthaltend die Ameisensäure-Amin-Addukte und das tertiäre Amin (Strom (7), wird dem Phasentrenngefäß (IV) zugeführt. Die Ameisensäure-Amin-Addukte werden im Phasentrenngefäß (IV) abgetrennt und der Destillationseinheit (V) als Strom (8) zugeführt, worin diese in freie Ameisensäure und tertiäres Amin thermisch gespalten werden. Die freie Ameisensäure wird als Kopfprodukt, Strom (10), abgezogen und der zweiphasige Sumpf aus der Destillationseinheit (V), enthaltend tertiäres Amin und nicht gespaltene Ameisensäure/Amin-Addukte, Strom (9), wird erneut dem Phasentrenngefäß (IV) zugeführt. Das tertiäre Amin, welches im Phasentrenngefäß (IV) abgetrennt wird, wird als Strom (11) der Extraktionseinheit (II) zugeführt, um den Katalysator zu extrahieren bzw. Teile von Strom (11) können direkt in den Hydrierreaktor (I) zurückgeführt werden, falls nicht alles Trialkylamin zur Extraktion benötigt wird.

Die Ausführungsform gemäß Figur 4 unterscheidet sich von den Ausführungsformen gemäß Figur 1 und 2 dadurch, dass ein Teil des zweiphasigen Sumpfgemisches (Strom (7)) dem Phasentrenngefäß IV zugeführt wird und ein Teil des zweiphasigen Sumpfgemisches (Strom (7)) der Destillationseinheit (V) zugeführt wird.

Die Figuren 5A, 5B und 5C zeigen schematisch jeweils unterschiedliche Varianten für die thermische Abtrennung von Wasser und polarem Lösungsmittel für die Ausführungsform nach Figur 3.

Hierbei zeigt Figur 5A die Abtrennung beider Ströme, d.h. des überwiegend polares Lösungsmittel enthaltenden Stromes (6) und des überwiegend Wasser enthaltenden Stromes (12) in einer einzigen Destillationskolonne, die eine Kolonne mit Seitenabzug auch eine Trennwandkolonne sein kann.

Figur 5B zeigt schematisch eine Ausführungsform mit einer 2-Kolonnen-Variante (Kolonnen (IIIa) und (IIIb)), wobei in der ersten Kolonne, IIIa, die leichter siedende Komponente, in der Regel das polare Lösungsmittel, und in der zweiten Kolonne, (IIIb), der ein Eduktstrom (5a) zugeführt wird, der von polarem Lösungsmittel abgereichert ist, die mittelsiedende Komponente, in der Regel das Wasser, als Strom (12), abgetrennt wird.

Figur 5C zeigt eine weitere Ausführungsform für die Abtrennung von Wasser und polarem Lösungsmittel, wobei in einer ersten Destillationskolonne (IIIc) zunächst die Phase (7) abgetrennt wird, enthaltend die Ameisensäure/Amin-Addukte sowie das tertiäre Amin, und anschließend ein Strom (5b) in einer zweiten Destillationskolonne (IIId) aufgetrennt wird, unter Erhalt eines überwiegend polares Lösungsmittel enthaltenden Stromes (6) und eines überwiegend Wasser enthaltenden Stromes (12).

## Patentansprüche

1. Verfahren zur Herstellung von Ameisensäure durch Umsetzung von Kohlendioxid (1) mit Wasserstoff (2) in einem Hydrierreaktor (I) in Gegenwart

    - eines Katalysators, enthaltend ein Element aus der 8., 9. oder 10. Gruppe des Periodensystems,
    - eines tertiären Amins, enthaltend mindestens 12 Kohlenstoffatome pro Molekül, sowie
    - eines polaren Lösungsmittels, enthaltend einen oder mehrere Monoalkohole, ausgewählt aus Methanol, Ethanol, Propanolen und Butanolen,

    unter Bildung von Ameisensäure/Amin-Addukten als Intermediaten, die anschließend thermisch gespalten werden,
    wobei ein tertiäres Amin eingesetzt wird, das einen um mindestens 5 °C höheren Siedepunkt als Ameisensäure

aufweist, und wobei
sich bei der Umsetzung im Hydrierreaktor (I) ein Reaktionsgemisch enthaltend das polare Lösungsmittel, die Ameisensäure/Amin-Addukte, das tertiäre Amin und den Katalysator, bildet, das aus dem Reaktor als Austrag (3) ausgetragen wird,
**dadurch gekennzeichnet, dass**
der Austrag (3) aus dem Hydrierreaktor (I), gegebenenfalls nach Zugabe von Wasser, direkt einer Extraktionseinheit (II) zugeführt wird und die Aufarbeitung des Austrags (3) die folgenden Verfahrensschritte umfasst:

1) Extraktion des Katalysators aus dem Austrag (3) aus dem Hydrierreaktor (I) in der Extraktionseinheit (II), wobei als Extraktionsmittel das gleiche tertiäre Amin, das in der Hydrierung eingesetzt wurde, eingesetzt wird, unter Erhalt eines Extrakts (4), der den Hauptteil des tertiären Amins und des Katalysators enthält, und der in den Hydrierreaktor (I) recycliert wird sowie eines Raffinats (5), das den Hauptteil des polaren Lösungsmittels und der Ameisensäure/Amin-Addukte enthält, und das in eine Destillationseinheit (III) weitergeleitet wird, worin
2) eine Destillation durchgeführt wird unter Erhalt eines Kopfstroms (6), der überwiegend das polare Lösungsmittel enthält, und der in den Hydrierreaktor (I) recycliert wird, sowie eines Sumpfstroms (7), der überwiegend die Ameisensäure/Amin-Addukte und das tertiäre Amin enthält, und welcher gemäß Verfahrensschritt
3) einem Phasentrenngefäß (IV) zugeführt wird, worin

a) eine Phasentrennung durchgeführt wird unter Erhalt einer Oberphase, enthaltend überwiegend das tertiäre Amin, die als Strom (11) in die Extraktionseinheit (II) als Extraktionsmittel für den Katalysator recycliert wird, und einer Unterphase, enthaltend überwiegend die Ameisensäure/Amin-Addukte, die als Strom (8) einer thermischen Spalteinheit (V) zugeführt wird, worin
b) eine thermische Spaltung durchgeführt wird unter Erhalt eines das tertiäre Amin enthaltenden Stroms (9), der in das Phasentrenngefäß (IV) zurückgeführt wird und eines die Ameisensäure enthaltenden Stroms (10),

oder gemäß Verfahrensschritt
4) einer thermischen Spalteinheit (V) zugeführt wird, worin

a) eine thermische Spaltung durchgeführt wird unter Erhalt eines die Ameisensäure enthaltenden Stroms (10) und eines das tertiäre Amin und die Ameisensäure/Amin-Addukte enthaltenden Stroms (9), der einem Phasentrenngefäß (IV) zugeführt wird, worin
b) eine Phasentrennung durchgeführt wird unter Erhalt einer Oberphase, enthaltend überwiegend das tertiäre Amin, die als Strom (11) in die Extraktionseinheit (II) als Extraktionsmittel für den Katalysator recycliert wird, und einer Unterphase, enthaltend überwiegend die Ameisensäure/Amin-Addukte, die als Strom (8) einer thermischen Spalteinheit (V) zugeführt wird.

**2.** Verfahren nach Anspruch 1, **dadurch gekennzeichnet, dass** ein Teilstrom des Sumpfstroms (7) gemäß Verfahrensschritt 3) der Sumpfstrom (7) im Übrigen gemäß Verfahrensschritt 4) aufgearbeitet wird.

**3.** Verfahren nach Anspruch 1 oder 2, **dadurch gekennzeichnet, dass** in Verfahrensschritt 2) eine fraktionierte Destillation durchgeführt wird unter Erhalt einer ersten Fraktion (6), die überwiegend das polare Lösungsmittel enthält, und die in den Hydrierreaktor (I) recycliert wird, einer zweiten Fraktion (12), die überwiegend Wasser enthält, und die in den Austrag (3) aus dem Hydrierreaktor (I) recycliert wird

**4.** Verfahren nach einem der Ansprüche 1 bis 3, **dadurch gekennzeichnet, dass** man als tertiäres Amin ein Amin der allgemeinen Formel (Ia)

$$NR^1R^2R^3 \qquad (Ia)$$

einsetzt, in der die Reste $R^1$ bis $R^3$ gleich oder verschieden sind und unabhängig voneinander einen unverzweigten oder verzweigten, acyclischen oder cyclischen, aliphatischen, araliphatischen oder aromatischen Rest mit jeweils 1 bis 6 Kohlenstoffatomen darstellen, wobei einzelne Kohlenstoffatome unabhängig voneinander auch durch eine Heterogruppe ausgewählt aus der Gruppe -O- und >N- substituiert sein können sowie zwei oder alle drei Reste unter Bildung einer mindestens jeweils vier Atome umfassenden Kette auch miteinander verbunden sein können, mit der Bedingung, dass das tertiäre Amin mindestens 12 Kohlenstoffatome pro Molekül enthält.

**5.** Verfahren nach Anspruch 4, **dadurch gekennzeichnet, dass** man als tertiäres Amin ein Amin der allgemeinen Formel (Ia) einsetzt, in der die Reste $R^1$ bis $R^3$ unabhängig voneinander ausgewählt sind aus der Gruppe $C_1$- bis $C_{12}$-Alkyl, $C_5$-bis $C_8$-Cycloalkyl, Benzyl und Phenyl.

**6.** Verfahren nach Anspruch 4, **dadurch gekennzeichnet, dass** man als tertiäres Amin ein gesättigtes Amin der allgemeinen Formel (Ia) einsetzt.

**7.** Verfahren nach Anspruch 4, **dadurch gekennzeichnet, dass** man als tertiäres Amin ein Amin der allgemeinen Formel (Ia) einsetzt, in der die Reste $R^1$ bis $R^3$ unabhängig voneinander ausgewählt sind aus $C_5$- und $C_6$-Alkyl.

**8.** Verfahren nach einem der Ansprüche 1 bis 7, **dadurch gekennzeichnet, dass** als polares Lösungsmittel ein Gemisch aus einem oder mehreren Monoalkoholen, ausgewählt aus Methanol, Ethanol, Propanolen und Butanolen mit Wasser eingesetzt wird.

**9.** Verfahren nach einem der Ansprüche 1 bis 8, **dadurch gekennzeichnet, dass** als polares Lösungsmittel ein Gemisch aus Methanol und/oder Ethanol mit Wasser eingesetzt wird.

**10.** Verfahren nach einem der Ansprüche 1 bis 7, **dadurch gekennzeichnet, dass** man als polares Lösungsmittel Methanol und/oder Ethanol einsetzt.

**11.** Verfahren nach einem der Ansprüche 1 bis 10, **dadurch gekennzeichnet, dass** das in Verfahrensschritt 1) erhaltene Raffinat (5), einen Wassergehalt, bezogen auf das Gesamtgewicht des Raffinats im Bereich von 0,1 bis 50 Gew.-%, bevorzugt im Bereich von 2 bis 30 Gew.-%, aufweist.

**12.** Verfahren nach einem der Ansprüche 1 bis 11, **dadurch gekennzeichnet, dass** der Katalysator ein homogener Katalysator ist.

**13.** Verfahren nach Anspruch 9, **dadurch gekennzeichnet, dass** der homogene Katalysator eine Komplexverbindung ist, enthaltend ein Element aus der 8., 9. oder 10. Gruppe des Periodensystems und mindestens eine Phosphingruppe mit mindestens einem unverzweigten oder verzweigten, acyclischen oder cyclischen aliphatischen Rest mit 1 bis 12 Kohlenstoffatomen, wobei einzelne Kohlenstoffatome auch durch >P- substituiert sein können.

**14.** Verfahren nach einem der Ansprüche 1 bis 13, **dadurch gekennzeichnet, dass** die Umsetzung im Hydrierreaktor (I) bei einer Temperatur im Bereich zwischen 20 und 200 °C und bei einem Druck im Bereich zwischen 0,2 und 30 MPa abs durchgeführt wird.

**15.** Verfahren nach einem der Ansprüche 1 bis 14, **dadurch gekennzeichnet, dass** die Extraktion bei Temperaturen im Bereich von 40 bis 80 °C durchgeführt wird.

## Claims

**1.** A process for preparing formic acid by reacting carbon dioxide (1) with hydrogen (2) in a hydrogenation reactor (I) in the presence of

- a catalyst comprising an element of group 8, 9 or 10 of the Periodic Table,
- a tertiary amine comprising at least 12 carbon atoms per molecule and
- a polar solvent comprising one or more monoalcohols selected from among methanol, ethanol, propanols and butanols,

to form formic acid/amine adducts as intermediates which are subsequently thermally dissociated,
where a tertiary amine having a boiling point which is at least 5°C higher than that of formic acid is used and a reaction mixture comprising the polar solvent, the formic acid/amine adducts, the tertiary amine and the catalyst is formed in the reaction in the hydrogenation reactor (I) and is discharged from the reactor as output (3), wherein
the output (3) from the hydrogenation reactor (I) is, optionally after addition of water, fed directly to an extraction unit (II) and the work-up of the output (3) comprises the following process steps:

1) extraction of the catalyst from the output (3) from the hydrogenation reactor (I) in the extraction unit (II),

where the same tertiary amine which was used in the hydrogenation is used as extractant, to give an extract (4) which comprises the major part of the tertiary amine and the catalyst and is recycled to the hydrogenation reactor (I) and also a raffinate (5) which comprises the major part of the polar solvent and the formic acid/amine adducts and is passed on to a distillation unit (III) in which
2) a distillation is carried out to give an overhead stream (6) which comprises predominantly the polar solvent and is recycled to the hydrogenation reactor (I) and also a bottom stream (7) which comprises predominantly the formic acid/amine adducts and the tertiary amine and is according to process step
3) fed to a phase separation vessel (IV) in which

a) a phase separation is carried out to give an upper phase which comprises predominantly the tertiary amine and is recycled as stream (11) to the extraction unit (II) as extractant for the catalyst and a lower phase which comprises predominantly the formic acid/amine adducts and is fed as stream (8) to a thermal dissociation unit (V) in which
b) a thermal dissociation is carried out to give a stream (9) which comprises the tertiary amine and is recirculated to the phase separation vessel (IV) and a stream (10) comprising the formic acid or according to process step

4) is fed to a thermal dissociation unit (V) in which

a) a thermal dissociation is carried out to give a stream (10) comprising the formic acid and a stream (9) which comprises the tertiary amine and the formic acid/amine adducts and is fed to a phase separation vessel (IV) in which
b) a phase separation is carried out to give an upper phase which comprises predominantly the tertiary amine and is recycled as stream (11) to the extraction unit (II) as extractant for the catalyst and a lower phase which comprises predominantly the formic acid/amine adducts and is fed as stream (8) to a thermal dissociation unit (V).

**2.** The process according to claim 1, wherein a substream of the bottom stream (7) is worked up as per process step 3) and the remainder of the bottom stream (7) is worked up as per process step 4).

**3.** The process according to claim 1 or 2, wherein, in process step 2), a fractional distillation is carried out to give a first fraction (6) which comprises predominantly the polar solvent and is recycled to the hydrogenation reactor (I) and a second fraction (12) which comprises predominantly water and is recycled to the output (3) from the hydrogenation reactor (I).

**4.** The process according to any of claims 1 to 3, wherein an amine of the general formula (Ia)

$$NR^1R^2R^3 \qquad (Ia)$$

where the radicals $R^1$ to $R^3$ are identical or different and are each, independently of one another, an unbranched or branched, acyclic or cyclic, aliphatic, araliphatic or aromatic radical having in each case from 1 to 6 carbon atoms, where individual carbon atoms can also, independently of one another, be substituted by a heterogroup selected from the group consisting of -O- and >N-and two or all three of the radicals can also be joined to one another to form a chain comprising at least four atoms, with the proviso that the tertiary amine comprises at least 12 carbon atoms per molecule, is used as tertiary amine.

**5.** The process according to claim 4, wherein an amine of the general formula (Ia) in which the radicals $R^1$ to $R^3$ are selected independently from the group consisting of $C_1$-$C_{12}$-alkyl, $C_5$-$C_8$-cycloalkyl, benzyl and phenyl is used as tertiary amine.

**6.** The process according to claim 4, wherein a saturated amine of the general formula (Ia) is used as tertiary amine.

**7.** The process according to claim 4, wherein an amine of the general formula (Ia) in which the radicals $R^1$ to $R^3$ are selected independently from among $C_5$- and $C_6$-alkyl is used as tertiary amine.

**8.** The process according to any of claims 1 to 7, wherein a mixture of one or more monoalcohols selected from among methanol, ethanol, propanols and butanols with water is used as polar solvent.

**9.** The process according to any of claims 1 to 8, wherein a mixture of methanol and/or ethanol with water is used as polar solvent.

**10.** The process according to any of claims 1 to 7, wherein methanol and/or ethanol is used as polar solvent.

**11.** The process according to any of claims 1 to 10, wherein the raffinate (5) obtained in process step 1) has a water content, based on the total weight of the raffinate, in the range from 0.1 to 50% by weight, preferably in the range from 2 to 30% by weight.

**12.** The process according to any of claims 1 to 11, wherein the catalyst is a homogeneous catalyst.

**13.** The process according to claim 9, wherein the homogeneous catalyst is a complex comprising an element of group 8, 9 or 10 of the Periodic Table and at least one phosphine group having at least one unbranched or branched, acyclic or cyclic aliphatic radical having from 1 to 12 carbon atoms, where individual carbon atoms can also be substituted by >P-.

**14.** The process according to any of claims 1 to 13, wherein the reaction in the hydrogenation reactor (I) is carried out at a temperature in the range from 20 to 200°C and a pressure in the range from 0.2 to 30 MPa abs.

**15.** The process according to any of claims 1 to 14, wherein the extraction is carried out at temperatures in the range from 40 to 80°C.

**Revendications**

**1.** Procédé pour la production d'acide formique par mise en réaction de dioxyde de carbone (1) avec de l'hydrogène (2) dans un réacteur d'hydrogénation (I) en présence

- d'un catalyseur, contenant un élément du $8^e$, $9^e$ ou $10^e$ groupe du système périodique,
- d'une amine tertiaire, comportant au moins 12 atomes de carbone par molécule, ainsi que
- d'un solvant polaire, contenant un ou plusieurs monoalcools, choisis parmi le méthanol, l'éthanol, les propanols et les butanols,

avec formation d'adduits acide formique/amine en tant que composés intermédiaires, qui sont ensuite scindés par voie thermique,
dans lequel on utilise une amine tertiaire qui présente un point d'ébullition supérieur d'au moins 5 °C à celui de l'acide formique, et dans lequel
lors de la réaction dans le réacteur d'hydrogénation (I) il se forme un mélange réactionnel contenant le solvant polaire, les adduits acide formique/amine, l'amine tertiaire et le catalyseur, qui est déchargé du réacteur en tant que courant de décharge (3),
**caractérisé en ce que**
le courant de décharge (3) provenant du réacteur d'hydrogénation (I), éventuellement après addition d'eau, est envoyé directement à une unité d'extraction (II) et le traitement final du courant de décharge (3) comprend les étapes de processus suivantes :

1) extraction du catalyseur à partir du courant de décharge (3) provenant du réacteur d'hydrogénation (I), dans l'unité d'extraction (II), en tant qu'agent d'extraction étant utilisée la même amine tertiaire que celle qui a été utilisée dans l'hydrogénation, avec obtention d'un extrait (4) qui contient la majeure partie de l'amine tertiaire et du catalyseur, et qui est recyclé dans le réacteur d'hydrogénation (I), ainsi que d'un raffinat (5) qui contient la majeure partie du solvant polaire et des adduits acide formique/amine, et qui est envoyé à son tour dans une unité de distillation (III), dans laquelle
2) est effectuée une distillation avec obtention d'un courant de tête (6) qui contient essentiellement le solvant polaire et qui est recyclé dans le réacteur d'hydrogénation (I), ainsi que d'un courant de pied (7) qui contient essentiellement les adduits acide formique/amine et l'amine tertiaire, et qui, selon l'étape de processus
3) est envoyé à un récipient de séparation de phases (IV), dans lequel

a) est effectuée une séparation de phases avec obtention d'une phase supérieure, contenant essentiellement l'amine tertiaire, qui est recyclée en tant que courant (11) dans l'unité d'extraction (II), en tant qu'agent

d'extraction pour le catalyseur, et d'une phase inférieure, contenant essentiellement les adduits acide formique/amine, qui est envoyée en tant que courant (8) à une unité de dissociation thermique (V), dans laquelle
b) est effectuée une dissociation thermique avec obtention d'un courant (9) contenant l'amine tertiaire, qui est renvoyé dans le récipient de séparation de phases (IV) et d'un courant (10) contenant l'acide formique, ou selon l'étape de processus

4) est envoyé à une unité de dissociation thermique(V), dans laquelle

a) est effectuée une dissociation thermique avec obtention d'un courant (10) contenant l'acide formique et d'un courant (9) contenant l'amine tertiaire et les adduits acide formique/amine, qui est envoyé à un récipient de séparation de phases (IV), dans lequel
b) est effectuée une séparation de phases avec obtention d'une phase supérieure, contenant essentiellement l'amine tertiaire, qui est recyclée en tant que courant (11) dans l'unité d'extraction (II), en tant qu'agent d'extraction pour le catalyseur, et d'une phase inférieure, contenant essentiellement les adduits acide formique/amine, qui est envoyée en tant que courant (8) à une unité de dissociation thermique (V).

**2.** Procédé selon la revendication 1, **caractérisé en ce qu'**un courant partiel du courant de pied (7) est soumis au traitement final selon l'étape de processus 3) et pour le reste le courant de pied (7) est soumis au traitement final selon l'étape de processus 4).

**3.** Procédé selon la revendication 1 ou 2, **caractérisé en ce que** dans l'étape de processus 2) on effectue une distillation fractionnée avec obtention d'une fraction (6), qui contient essentiellement le solvant polaire, et qui est recyclée dans le réacteur d'hydrogénation (I), d'une deuxième fraction (12), qui contient essentiellement de l'eau, et qui est recyclée dans le courant de décharge (3) provenant du réacteur d'hydrogénation (I).

**4.** Procédé selon l'une quelconque des revendications 1 à 3, **caractérisé en ce qu'**on utilise comme amine tertiaire une amine de formule générale (Ia)

$$NR^1R^2R^3 \qquad (Ia)$$

dans laquelle les radicaux $R^1$ à $R^3$ sont identiques ou différents et représentent chacun indépendamment un radical aliphatique acyclique ramifié ou non ramifié, ou cyclique, araliphatique ou aromatique, ayant chaque fois de 1 à 6 atomes de carbone, des atomes de carbone individuels pouvant, indépendamment les uns des autres, être également remplacés par un hétéroatome choisi dans le groupe constitué par -O- et >N- et deux ou tous les trois radicaux pouvant également être liés entre eux avec formation d'une chaîne comprenant au moins chaque fois quatre atomes, étant entendu que l'amine tertiaire contient au moins 12 atomes de carbone par molécule.

**5.** Procédé selon la revendication 4, **caractérisé en ce qu'**on utilise comme amine tertiaire une amine de formule générale (Ia), dans laquelle les radicaux $R^1$ à $R^3$ sont choisis chacun indépendamment dans le groupe constitué par des groupes alkyle en $C_1$-$C_{12}$, cycloalkyle en $C_5$-$C_8$, benzyle et phényle.

**6.** Procédé selon la revendication 4, **caractérisé en ce qu'**on utilise comme amine tertiaire une amine saturée de formule générale (Ia).

**7.** Procédé selon la revendication 4, **caractérisé en ce qu'**on utilise comme amine tertiaire une amine de formule générale (Ia), dans laquelle les radicaux $R^1$ à $R^3$ sont choisis chacun indépendamment parmi des groupes alkyle en $C_5$ et $C_6$.

**8.** Procédé selon l'une quelconque des revendications 1 à 7, **caractérisé en ce qu'**on utilise comme solvant polaire un mélange d'un ou de plusieurs monoalcools, choisis parmi le méthanol, l'éthanol, les propanols et les butanols, avec l'eau.

**9.** Procédé selon l'une quelconque des revendications 1 à 8, **caractérisé en ce qu'**on utilise comme solvant polaire un mélange de méthanol et/ou d'éthanol avec l'eau.

**10.** Procédé selon l'une quelconque des revendications 1 à 7, **caractérisé en ce qu'**on utilise comme solvant polaire le méthanol et/ou l'éthanol.

**11.** Procédé selon l'une quelconque des revendications 1 à 10, **caractérisé en ce que** le raffinat (5) obtenu dans l'étape de processus 1) présente une teneur en eau, par rapport au poids total du raffinat, dans la plage de 0,1 à 50 % en poids, de préférence dans la plage de 2 à 30 % en poids.

**12.** Procédé selon l'une quelconque des revendications 1 à 11, **caractérisé en ce que** le catalyseur est un catalyseur homogène.

**13.** Procédé selon la revendication 9, **caractérisé en ce que** le catalyseur homogène est un composé complexe, contenant un élément du $8^e$, $9^e$ ou $10^e$ groupe du système périodique et au moins un groupe phosphine comportant au moins un radical aliphatique ramifié ou non ramifié, acyclique ou cyclique, ayant de 1 à 12 atomes de carbone, dans lequel des atomes de carbone individuels peuvent également être substitués par >P-.

**14.** Procédé selon l'une quelconque des revendications 1 à 13, **caractérisé en ce que** la réaction dans le réacteur d'hydrogénation (I) est effectuée à une température dans la plage comprise entre 20 et 200 °C et sous une pression dans la plage comprise entre 0,2 et 30 MPa (absolue).

**15.** Procédé selon l'une quelconque des revendications 1 à 14, **caractérisé en ce qu'**on effectue l'extraction à des températures dans la plage de 40 à 80 °C.

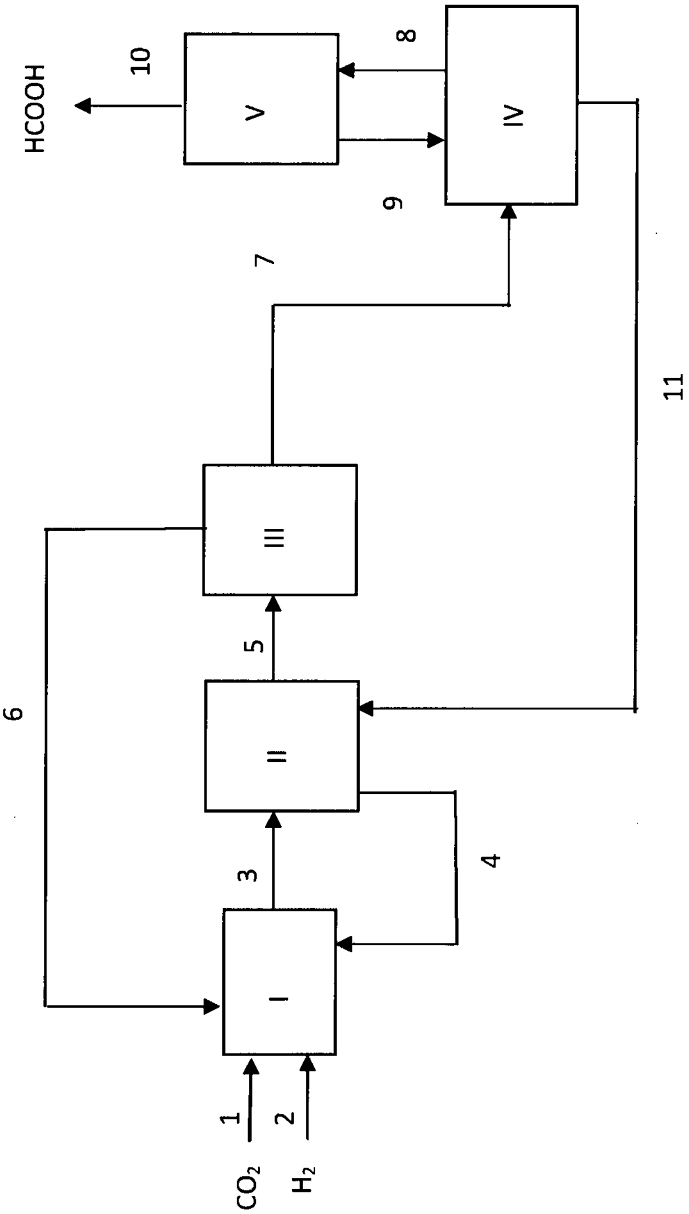


Figur 1

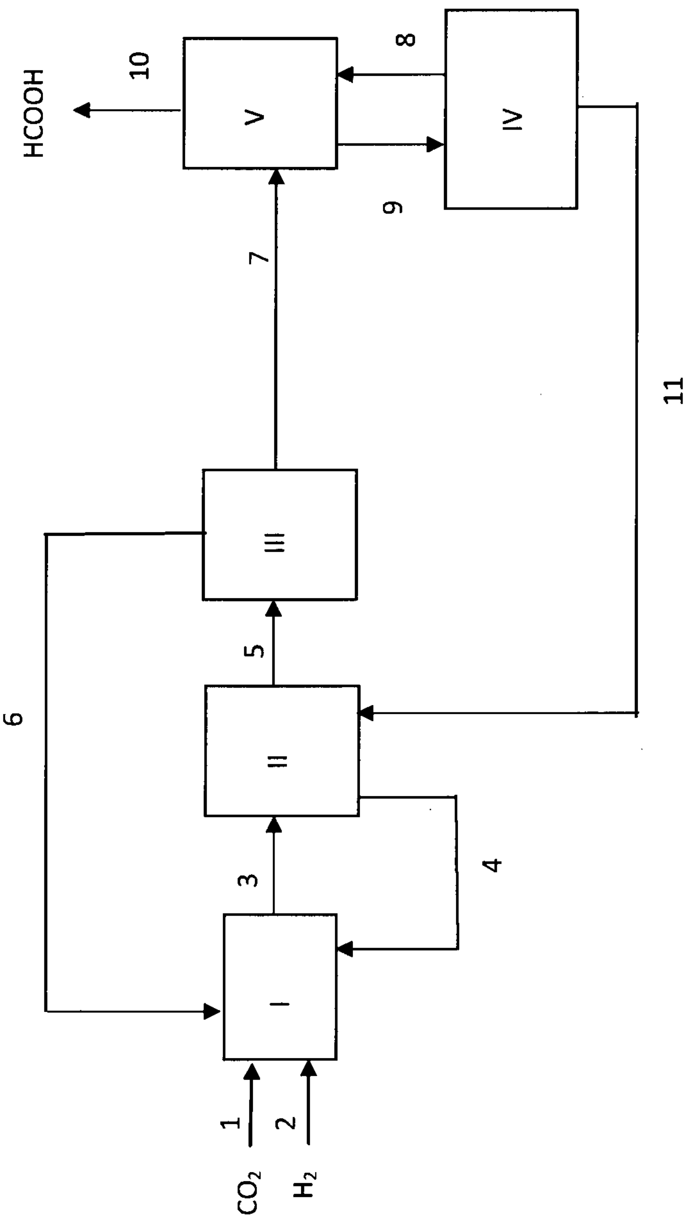


Figur 2

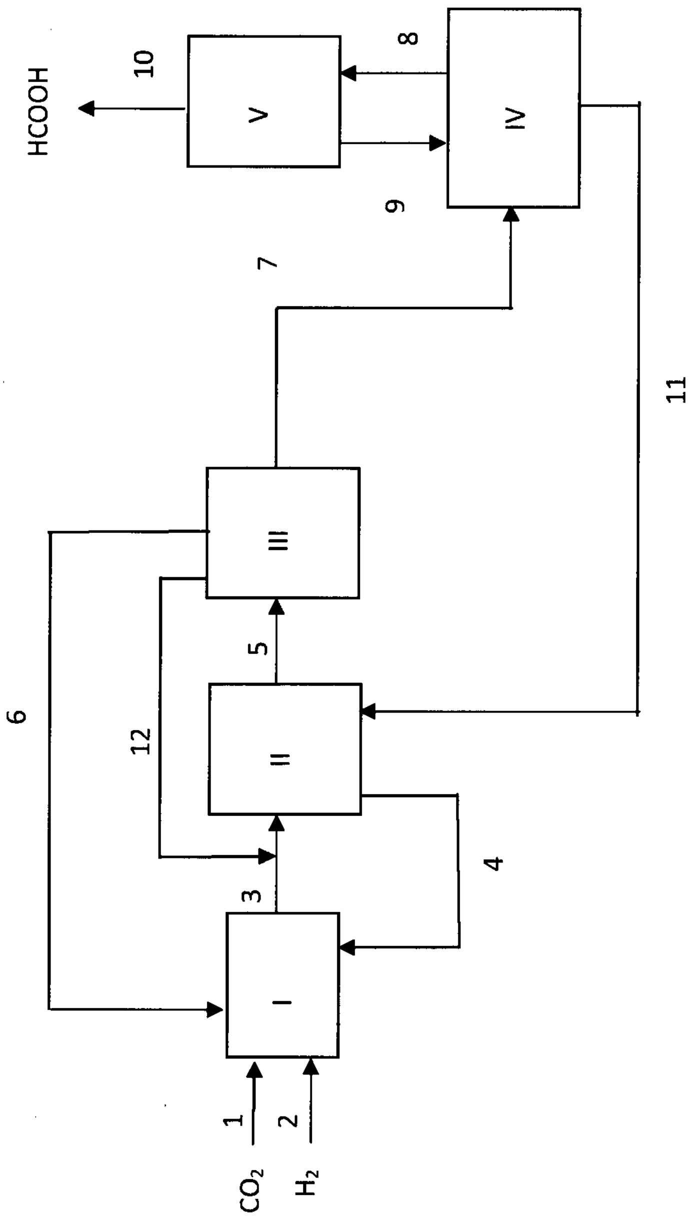


Figur 3

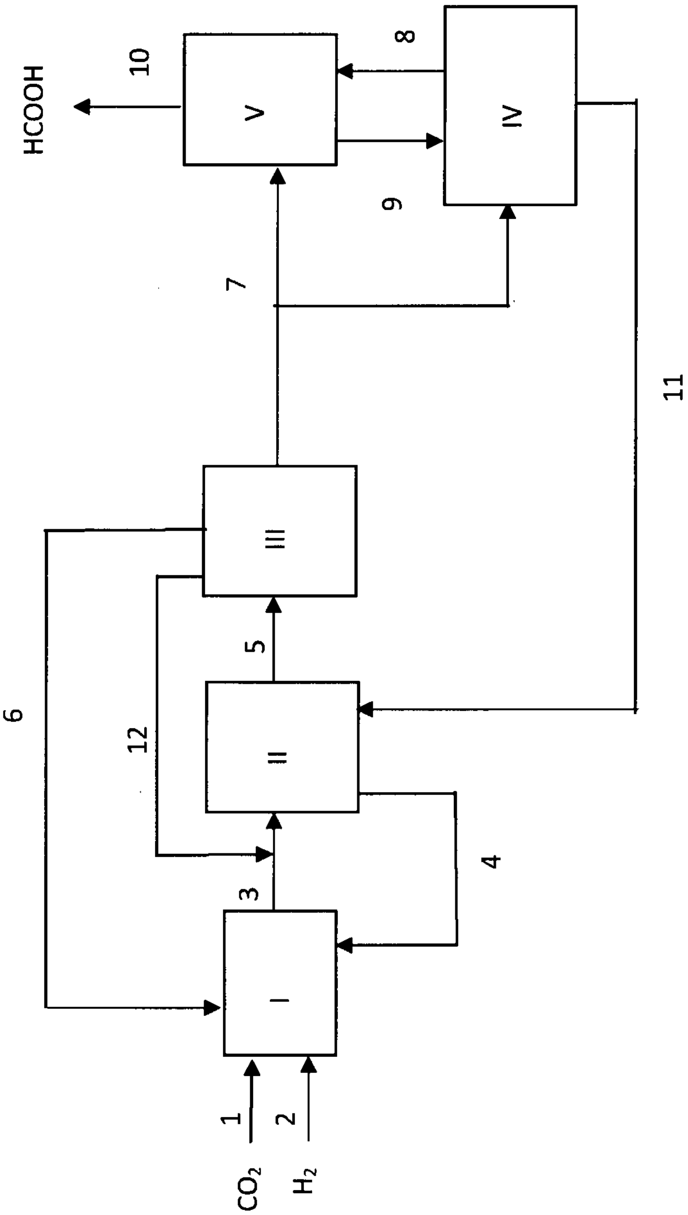


Figur 4

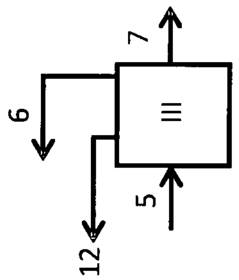


Figur 5A

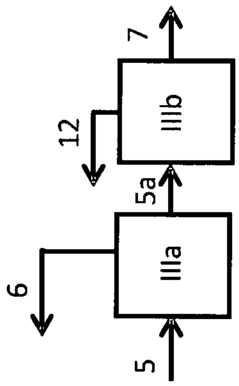


Figur 5B

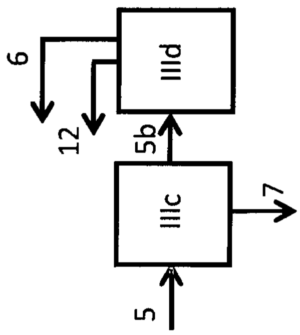


Figur 5C

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008116799 A
- EP 0095321 A
- EP 0151510 A
- EP 0181078 A
- EP 0126524 A
- EP 0329337 A
- EP 0357243 A
- DE 4431233 A
- WO 2006021411 A
- EP 181078 B1
- EP 357243 B1


### In der Beschreibung aufgeführte Nicht-Patentliteratur


- **W. LEITNER.** *Angewandte Chemie,* 1995, vol. 107, 2391-2405
- **P. G. JESSOP ; T. IKARIYA ; R. NOYORI.** *Chemical Reviews,* 1995, vol. 95, 252-272
- **C. FELLAY ; N. YAN ; P.J. DYSON ; G. LAURENCZY.** *Chem. Eur. J.,* 2009, vol. 15, 3752-3760
- **C. FELLAY ; P.J. DYSON ; G. LAURENCZY.** *Angew. Chem.,* 2008, vol. 120, 4030-4032
- **B. LOGES ; A. BODDIEN ; H. JUNGE ; M. BELLER.** *Angew. Chem.,* 2008, vol. 120, 4026-4029
- **F. JOÓ.** *ChemSusChem,* 2008, vol. 1, 805-808
- **S. ENTHALER.** *ChemSusChem,* 2008, vol. 1, 801-804
- **S. FUKUZUMI ; T. KOBAYASHI ; T. SUENOBU.** *ChemSusChem,* 2008, vol. 1, 827-834
- **A. BODDIEN ; B. LOGES ; H. JUNGE ; M. BELLER.** *ChemSusChem,* 2008, vol. 1, 751-758
- **P.G. JESSOP ; T. IKARIYA ; R. NOYORI.** *Chem. Rev.,* 1995, vol. 95, 259-272
- **P.G. JESSOP ; F. JOÓ, C.C. TAI.** *Coord. Chem. Rev.,* 2004, vol. 248, 2425-2442
- Homogeneous Hydrogenation of Carbon Dioxide. **P.G. JESSOP.** The Handbook of Homogeneous Hydrogenation. Wiley-VCH, 2007, vol. 1, 489-511
- Reactor Types and Their Industrial Applications. **K.D. HENKEL.** Ullmann's Encyclopedia of Industrial Chemistry. Wiley-VCH Verlag GmbH & Co. KGaA, 2005